# EUROPEAN PATENT SPECIFICATION

(11) **EP 2 041 082 B1**
(45) Date of publication and mention of the grant of the patent: **30.09.2009**
(21) Application number: 07786764.6
(22) Date of filing: 20.06.2007
(51) Int. Cl.: C07D 207/06, A61K 31/401, A61P 25/18

(54) **PYRROLIDINE DERIVATIVES HAVING ACTIVITY AT THE GLYT1 TRANSPORTER**
PYRROLIDINDERIVATE MIT WIRKUNG AM GLYT1-TRANSPORTER
COMPOSÉS AYANT UNE ACTIVITÉ AU NIVEAU DU TRANSPORTEUR GLYT1

(30) Priority: 22.06.2006 GB 0612420
(43) Date of publication of application: 01.04.2009
(73) Proprietor: Glaxo Group Limited, Greenford Middlesex UB6 0NN (GB)
(72) Inventor: ANDERTON, Clare, Louise, Stevenage Hertfordshire SG1 2NY (GB); CLAPHAM, David, Harlow Essex CM19 5AW (GB); KEEL, Trevor, Raymond,, Stevenage Hertfordshire SG1 2NY (GB); KINDON, Leanda, Jane, Stevenage Hertfordshire SG1 2NY (GB)
(74) Representative: Kondo, Rie
(86) International application number: PCT/EP2007/056106
(87) International publication number: WO 2007/147831

(56) References cited:
- WO-A-03/055478
- WO-A-20/05037785
- WO-A-20/06067423

## Description

The present invention relates to novel salts of 2-(methyloxy)-*N*-[2-methyl-1-phenyl-2-(1-pyrrolidinyl)propyl]-4,6-bis(trifluoromethyl)benzamide and solvates thereof, pharmaceutical formulations, processes for their preparation, and their use in medicine.

Molecular cloning has revealed the existence in mammalian brains of two classes of glycine transporters, termed GlyT1 and GlyT2. GlyT1 is found predominantly in the forebrain and its distribution corresponds to that of glutamatergic pathways and NMDA receptors (Smith, et al., Neuron, 8, 1992: 927-935). Molecular cloning has further revealed the existence of three variants of GlyT1, termed GlyT-la, GlyT-1b and GlyT-1c (Kim et al., Molecular Pharmacology, 45, 1994: 608-617), each of which displays a unique distribution in the brain and peripheral tissues. The variants arise by differential splicing and exon usage, and differ in their N-terminal regions. GlyT2, in contrast, is found predominantly in the brain stem and spinal cord, and its distribution corresponds closely to that of strychnine-sensitive glycine receptors (Liu et al., J. Biological Chemistry, 268, 1993: 22802-22808; Jursky and Nelson, J. Neurochemistry, 64, 1995 : 1026-1033). Another distinguishing feature of glycine transport mediated by GlyT2 is that it is not inhibited by sarcosine as is the case for glycine transport mediated by GlyT1. These data are consistent with the view that, by regulating the synaptic levels of glycine, GlyT1 and GlyT2 selectively influence the activity of NMDA receptors and strychnine-sensitive glycine receptors, respectively.

NMDA receptors are critically involved in memory and learning (Rison and Staunton, Neurosci. Biobehav. Rev., 19 533-552 (1995); Danysz et al, Behavioral Pharmacol., 6 455-474 (1995)); and, furthermore, decreased function of NMDA-mediated neurotransmission appears to underlie, or contribute to, the symptoms of schizophrenia (Olney and Farber, Archives General Psychiatry, 52, 998-1007 (1996). Thus, agents that inhibit GlyT1 and thereby increase glycine activation of NMDA receptors can be used as novel antipsychotics and anti-dementia agents, and to treat other diseases in which cognitive processes are impaired, such as attention deficit disorders and organic brain syndromes. Conversely, over-activation of NMDA receptors has been implicated in a number of disease states, in particular the neuronal death associated with stroke and possibly neurodegenerative diseases, such as Alzheimer's disease, multi-infarct dementia, AIDS dementia, Huntington's disease, Parkinson's disease, amyotrophic lateral sclerosis or other conditions in which neuronal cell death occurs, such as stroke or head trauma. Coyle & Puttfarcken, Science, 262, 689-695 (1993); Lipton and Rosenberg, New Engl. J. of Medicine, 330, 613-622 (1993); Choi, Neuron, 1, 623-634 (1988). Thus, pharmacological agents that increase the activity of GlyT1 will result in decreased glycine-activation of NMDA receptors, which activity can be used to treat these and related disease states. Similarly, drugs that directly block the glycine site of the NMDA receptors can be used to treat these and related disease states.

2-(methyloxy)-*N*-[2-methyl-1-phenyl-2-(1-pyrrolidinyl)propyl]-4,6-bis(trifluoromethyl)benzamide and its hydrochloride salt are disclosed in WO2006067423 as being glycine transport inhibitors and useful in the manufacture of medicaments for treating neurological and neuropsychiatric disorders, in particular psychoses, dementia or attention deficit disorder.

The structure of 2-(methyloxy)-*N*-[2-methyl-1-phenyl-2-(1-pyrrolidinyl)propyl]-4,6-bis(trifluoromethyl)benzamide is indicated below:

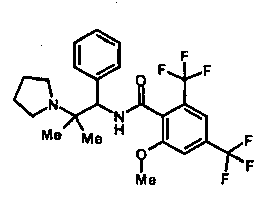

The compound of can be prepared, for example, by reacting 2,4-ditrifluoromethyl-6-methoxy-benzoic acid and [2-methyl-1-phenyl-2-(1-pyrrolidinyl)propyl]amine in an appropriate solvent such as DMF. As 2-(methyloxy)-*N*-[2-methyl-1-phenyl-2-(1-pyrrolidinyl)propyl]-4,6-bis(trifluoromethyl)benzamide is a chiral molecule, the (+) form and the (-) may be prepared by stereospecific synthesis and/or by resolution of the final product or any intermediate.

For use in medicine there exists a need for the compound to be prepared in a form suitable for ease of isolation in large scale manufacture and ease of formulating into an acceptable product for administration to patients. It is difficult to predict the physical characteristics of any particular salt of a compound and small differences in physical properties may equate to large savings in the manufacture and formulation of a pharmaceutical product containing the compound.

The present invention provides 2-(methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate, which may be used as alternatives to the free base and the hydrochloride salt of 2-(methyloxy)-*N*-[2-methyl-1-phenyl-2-(1-pyrrolidinyl)propyl]-4,6-bis(trifluoromethyl)benzamide for therapeutic administration or as an intermediate in the preparation of other salts.

The crystallinity, thermal properties, stability and hygroscopicity of both 2-(methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate are at acceptable levels for commercial use.

Therefore, the present invention provides 2-(methyloxy)-*N*-[2-methyl-1-phenyl-2-(1-pyrrolidinyl)propyl]-4,6-bis(trifluoromethyl)benzamide succinate or a solvate thereof.

The present invention also provides 2-(methyloxy)-*N*-[2-methyl-1-phenyl-2-(1-pyrrolidinyl)propyl]-4,6-bis(trifluoromethyl)benzamide napadisylate or a solvate thereof.

It should be noted that "napadisylate" is interchangeable with "napadisilate" and "naphthalenedisulfonate". Similarly the term "napadisylic acid" is interchangeable with "naphthalenedisulfonic acid".

As used herein, the term "solvate" refers to a complex of variable stoichiometry formed by a solute (in this invention, 2-(methyloxy)-*N*-[2-methyl-1-phenyl-2-(1-pyrrolidinyl)propyl]-4,6-bis(trifluoromethyl)benzamide succinate or napadisylate) and a solvent. Such solvents for the purpose of the invention may not interfere with the biological activity of the solute. Examples of suitable solvents include, but are not limited to, water, methanol, ethanol and acetic acid. In one embodiment, the solvent used is a pharmaceutically acceptable solvent. Examples of suitable pharmaceutically acceptable solvents include water, ethanol and acetic acid. In one embodiment, the solvent used is water.

2-(Methyloxy)-*N*-[2-methyl-1-phenyl-2-(1-pyrrolidinyl)propyl]-4,6-bis(trifluoromethyl)benzamide exists in two stereoisomeric forms, owing to the presence of an asymmetric carbon, indicated below with a star:

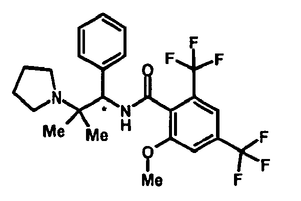

The enantiomers may be separated by chiral high-performance liquid chromatography or other appropriate means, or may be obtained by stereospecific synthesis, using any suitable method known in the art. See, for example, Stereochemistry of Organic Compounds by E. L. Eliel, S. H. Wilen, and L. N. Mander (Wiley-Interscience, 1994).

In one embodiment, an optically pure enantiomer is provided. The term "optically pure enantiomer" means that the compound contains greater than about 90% of the desired isomer by weight. In one embodiment, the compound contains greater than about 95% of the desired isomer by weight. In one embodiment, the compound contains greater than about 99% of the desired isomer by weight, said weight percent based upon the total weight of the isomer(s) of the compound.

The present invention includes the succinate and the napadisylate of both possible enantiomers of 2-(methyloxy)-*N*-[2-methyl-1-phenyl-2-(1-pyrrolidinyl)propyl]-4,6-bis(trifluoromethyl)benzamide, ie:
(R)-(-)-2-(methyloxy)-*N*-[2-methyl-1-phenyl-2-(1-pyrrolidinyl)propyl]-4,6-bis(trifluoromethyl)benzamide and
(S)-(+)-2-(methyloxy)-*N*-[2-methyl-1-phenyl-2-(1-pyrrolidinyl)propyl]-4,6-bis(trifluoromethyl)benzamide;
as well as any mixtures thereof, such as the racemic mixture: (±)-2-(methyloxy)-*N*-[2-methyl-1-phenyl-2-(1-pyrrolidinyl)propyl]-4,6-bis(trifluoromethyl)benzamide.

The present invention encompasses the succinate and the napadisylate salts of racemic compounds and that of the individual enantiomers as well.

In one embodiment, stereochemical isomers enriched in configuration (R)-(-)-2-(methyloxy)-*N*-[2-methyl-1-phenyl-2-(1-pyrrolidinyl)propyl]-4,6-bis(trifluoromethyl)benzamide succinate or R-(-)-2-(methyloxy)-*N*-[2-methyl-1-phenyl-2-(1-pyrrolidinyl)propyl]-4,6-bis(trifluoromethyl)benzamide napadisylate correspond to at least 90% enantiomeric excess. In another embodiment, the isomers correspond to at least 95% enantiomeric excess. In another embodiment, the isomers correspond to at least 99% enantiomeric excess.

In one embodiment, stereochemical isomers enriched in configuration S-(+)-2-(methyloxy)-*N*-[2-methyl-1-phenyl-2-(1-pyrrolidinyl)propyl]-4,6-bis(trifluoromethyl)benzamide succinate or S-(+)-2-(methyloxy)-*N*-[2-methyl-1-phenyl-2-(1-pyrrolidinyl)propyl]-4,6-bis(trifluoromethyl)benzamide napadisylate correspond to at least 90% enantiomeric excess. In another embodiment, the isomers correspond to at least 95% enantiomeric excess. In another embodiment, the isomers correspond to at least 99% enantiomeric excess.

Furthermore, naphthalenedisulfonic acid exists in several isomeric forms, such as 1,5-naphthalenedisulfonic acid and 1,3-naphthalenedisulfonic. The present invention encompasses all salts made from all forms of naphthalenedisulfonic acid, including 1,5-naphthalenedisulfonic acid and 1,3-naphthalenedisulfonic acid.

In one embodiment, the present invention provides 2-(methyloxy)-*N*-[2-methyl-1-phenyl-2-(1-pyrrolidinyl)propyl]-4,6-bis(trifluoromethyl)benzamide-1,5-napadisylate.

In another embodiment, the present invention provides 2-(methyloxy)-*N*-[2-methyl-1-phenyl-2-(1-pyrrolidinyl)propyl]-4,6-bis(trifluoromethyl)benzamide-1,3-napadisylate.

The 2-(methyloxy)-*N*-[2-methyl-1-phenyl-2-(1-pyrrolidinyl)propyl]-4,6-bis(trifluoromethyl)benzamide succinate or the napadisylate may be prepared by contacting appropriate stoichiometric amounts of the free base with succinic acid or napadisylic acid. In one embodiment, the base is in solution. In another embodiment, both are in solution. The present invention includes within its scope all possible stoichiometric and non-stoichiometric forms of 2-(methyloxy)-*N*-[2-methyl-1-phenyl-2-(1-pyrrolidinyl)propyl]-4,6-bis(trifluoromethyl)benzamide succinate and napadisylate. Thus the present invention includes:
- salts of 2-(methyloxy)-*N*-[2-methyl-1-phenyl-2-(1-pyrrolidinyl)propyl]-4,6-bis(trifluoromethyl)benzamide with succinic acid in molar ratio of 1:1 (which would give a monosuccinate salt);
- salts of 2-(methyloxy)-*N*-[2-methyl-1-phenyl-2-(1-pyrrolidinyl)propyl]-4,6-bis(trifluoromethyl)benzamide with succinic acid in molar ratio of 2:1 (which would give a hemisuccinate salt);
- salts of 2-(methyloxy)-*N*-[2-methyl-1-phenyl-2-(1-pyrrolidinyl)propyl]-4,6-bis(trifluoromethyl)benzamide with napadisylic acid in molar ratio of 1:1 (which would give a mononapadisylate salt); and
- salts of 2-(methyloxy)-*N*-[2-methyl-1-phenyl-2-(1-pyrrolidinyl)propyl]-4,6-bis(trifluoromethyl)benzamide with napadisylic acid in molar ratio of 2:1 (which would give a heminapadisylate salt).
In one embodiment, there is provided 2-(methyloxy)-*N*-[2-methyl-1-phenyl-2-(1-pyrrolidinyl)propyl]-4,6-bis(trifluoromethyl)benzamide succinate or a solvate thereof in which the ratio of 2-(methyloxy)-*N*-[2-methyl-1-phenyl-2-(1-pyrrolidinyl)propyl]-4,6-bis(trifluoromethyl)benzamide to succinic acid (by mole) is 1:1.

In one embodiment, there is provided 2-(methyloxy)-*N*-[2-methyl-1-phènyl-2-(1-pyrrolidinyl)propyl]-4,6-bis(trifluoromethyl)benzamide-1,5-napadisylate or a solvate thereof in which the ratio of 2-(methyloxy)-*N*-[2-methyl-1-phenyl-2-(1-pyrrolidinyl)propyl]-4,6-bis(trifluoromethyl)benzamide to napadisylic acid (by mole) is 2:1 (ie 2-(methyloxy)-*N*-[2-methyl-1-phenyl-2-(1-pyrrolidinyl)propyl]-4,6-bis(trifluoromethyl)benzamide-1,5-heminapadisylate or a solvate thereof).

Thus, the terms "2-(methyloxy)-*N*-[2-methyl-1-phenyl-2-(1-pyrrolidinyl)propyl]-4,6-bis(trifluoromethyl)benzamide succinate" and "2-(methyloxy)-*N*-[2-methyl-1-phenyl-2-(1-pyrrolidinyl)propyl]-4,6-bis(trifluoromethyl)benzamide napadisylate" as used herein include:
- (±)-2-(methyloxy)-*N*-[2-methyl-1-phenyl-2-(1-pyrrolidinyl)propyl]-4,6-bis(trifluoromethyl)benzamide hemisuccinate;
- (±)-2-(methyloxy)-*N*-[2-methyl-1-phenyl-2-(1-pyrrolidinyl)propyl]-4,6-bis(trifluoromethyl)benzamide monosuccinate;
- S-(+)-2-(methyloxy)-*N*-[2-methyl-1-phenyl-2-(1-pyrrolidinyl)propyl]-4,6-bis(trifluoromethyl)benzamide hemisuccinate;
- S-(+)-2-(methyloxy)-*N*-[2-methyl-1-phenyl-2-(1-pyrrolidinyl)propyl]-4,6-bis(trifluoromethyl)benzamide monosuccinate;
- R-(-)-2-(methyloxy)-*N*-[2-methyl-1-phenyl-2-(1-pyrrolidinyl)propyl]-4,6-bis(trifluoromethyl)benzamide hemisuccinate;
- R-(-)2-(methyloxy)-*N*-[2-methyl-1-phenyl-2-(1-pyrrolidinyl)propyl]-4,6-bis(trifluoromethyl)benzamide monosuccinate;
- (±)-2-(methyloxy)-*N*-[2-methyl-1-phenyl-2-(1-pyrrolidinyl)propyl]-4,6-bis(trifluoromethyl)benzamide-1,3-heminapadisylate;
- (±)-2-(methyloxy)-*N*-[2-methyl-1-phenyl-2-(1-pyrrolidinyl)propyl]-4,6-bis(trifluoromethyl)benzamide-1,3-mononapadisylate;
- (±)-2-(methyloxy)-*N*-[2-methyl-1-phenyl-2-(1-pyrrolidinyl)propyl]-4,6-bis(trifluoromethyl)benzamide-1,5-heminapadisylate;
- (±)-2-(methyloxy)-*N*-[2-methyl-1-phenyl-2-(1-pyrrolidinyl)propyl]-4,6-bis(trifluoromethyl)benzamide-1,5-mononapadisylate;
- S-(+)-2-(methyloxy)-*N*-[2-methyl-1-phenyl-2-(1-pyrrolidinyl)propyl]-4,6-bis(trifluoromethyl)benzamide-1,3-heminapadisylate;
- S-(+)-2-(methyloxy)-*N*-[2-methyl-1-phenyl-2-(1-pyrrolidinyl)propyl]-4,6-bis(trifluoromethyl)benzamide-1,3-mononapadisylate;
- S-(+)-2-(methyloxy)-*N*-[2-methyl-1-phenyl-2-(1-pyrrolidinyl)propyl]-4,6-bis(trifluoromethyl)benzamide-1,5-heminapadisylate;
- S-(+)-2-(methyloxy)-*N*-[2-methyl-1-phenyl-2-(1-pyrrolidinyl)propyl]-4,6-bis(trifluoromethyl)benzamide-1,5-mononapadisylate;
- R-(-)2-(methyloxy)-*N*-[2-methyl-1-phenyl-2-(1-pyrrolidinyl)propyl]-4,6-bis(trifluoromethyl)benzamide-1,3-heminapadisylate;
- R-(-)-2-(methyloxy)-*N*-[2-methyl-1-phenyl-2-(1-pyrrolidinyl)propyl]-4,6-bis(trifluoromethyl)benzamide-1,3-mononapadisylate;
- R-(-)-2-(methyloxy)-*N*-[2-methyl-1-phenyl-2-(1-pyrrolidinyl)propyl]-4,6-bis(trifluoromethyl)benzamide-1,5-heminapadisylate;
- R-(-)-2-(methyloxy)-*N*-[2-methyl-1-phenyl-2-(1-pyrrolidinyl)propyl]-4,6-bis(trifluoromethyl)benzamide-1,5-mononapadisylate; and
- a mixture of one or more of the above.

In one embodiment of the present invention, the succinate and the napadisylate is substantially free of alternative salt, free base or impurity. By "substantially free" is meant containing less than 10%, such as less than 5%, or less than 2%, of impurity. The impurity may be other compounds or other salts or solvates of 2-(methyloxy)-*N*-[2-methyl-1-phenyl-2-(1-pyrrolidinyl)propyl]-4,6-bis(trifluoromethyl)benzamide.

Depending on the solvent from which the succinate or the napadisylate is recovered, the succinate or the napadisylate may be obtained as a solvate. Such a solvate also forms one aspect of the present invention. In one embodiment, the solvate is a pharmaceutically acceptable solvate. A suitable solvate is a hydrate. In one embodiment, 2-(methyloxy)-*N-*[2-methyl-1-phenyl-2-(1-pyrrolidinyl)propyl]-4,6-bis(trifluoromethyl)benzamide succinate which is a 1:1 hydrate, or 2-(methyloxy)-*N*-[2-methyl-1-phenyl-2-(1-pyrrolidinyl)propyl]-4,6-bis(trifluoromethyl)benzamide napadisylate which is a 1:1 hydrate, is provided.

The present invention encompasses 2-(methyloxy)-*N*-[2-methyl-1-phenyl-2-(1-pyrrolidinyl)propyl]-4,6-bis(trifluoromethyl)benzamide succinate or the napadisylate or a solvate thereof isolated in pure form or when admixed with other materials.

Therefore, in one aspect there is provided 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, in isolated form.

In another aspect there is provided 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, in pure form. In one embodiment, 2-(methyloxy)-*N*-[2-methyl-1-phenyl-2-(1-pyrrolidinyl)propyl]-4,6-bis(trifluoromethyl)benzamide succinate or 2-(methyloxy)-*N*-[2-methyl-1-phenyl-2-(1-pyrrolidinyl)propyl]-4,6-bis(trifluoromethyl)benzamide napadisylate is greater than 90% pure, such as greater than 95% pure, or greater than 98% pure.

The compounds of the present invention may have the ability to crystallise in more than one form. This is a characteristic known as polymorphism, and it is understood that such polymorphic forms ("polymorphs") are within the scope of the present invention. Polymorphism generally can occur as a response to changes in temperature or pressure or both and can also result from variations in the crystallisation process. Polymorphs can be distinguished by various physical characteristics known in the art such as x-ray diffraction patterns, solubility, and melting point.

In a further aspect there is provided 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, in crystalline form. In one embodiment, there is provided 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, in substantially crystalline form.

As used herein, the term "substantially crystalline form" means that it is substantially free of amorphous form 2-(methyloxy)-*N*-[2-methyl-1-phenyl-2-(1-pyrrolidinyl)propyl]-4,6-bis(trifluoromethyl)benzamide succinate. By "substantially free" is meant containing less than 50% of the amorphous form, preferably less than 20% of the amorphous form, more preferably less than 10% of the amorphous form, more preferably less than 5% of the amorphous form, even more preferably less than 2% of the amorphous form, most preferably less than 1% of the amorphous form.

In a still further aspect there is provided 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, in polymorphic form(s).

The present invention also provides 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, when admixed with other material, for example another salt and/or solvate of 2-(methyloxy)-*N*-[2-methyl-1-phenyl-2-(1-pyrrolidinyl)propyl]-4,6-bis(trifluoromethyl)benzamide.

Most commonly used solvents are suitable for mobilising the free base, for example alcohols such as ethanol, ketones such as acetone, halogenated hydrocarbons such as dichloromethane, and ethers such as tetrahydrofuran. The acids may be added as a solid, as an aqueous solution, or as a solution in an organic solvent such as ethanol, or water, methanol, propan-2-ol, or acetone.

For the preparation of the succinate or the napadisylate, the concentration of 2-(methyloxy)-*N*-[2-methyl-1-phenyl-2-(1-pyrrolidinyl)propyl]-4,6-bis(trifluoromethyl)benzamide base is, in one embodiment in the range 3 to 25% weight/volume, such as in the range 5 to 15% . The concentration of succinic acid or napadisylic acid when used in solution may be in one embodiment in the range 0.5 to 10 molar, such as for example between 5 to 10 molar.

The salts may be isolated in solid form by conventional means from a solution thereof obtained as above. For example, a non-crystalline salt may be prepared by precipitation from solution, spray drying and freeze drying of solutions, evaporating a solution to a glass, or vacuum drying of oils, or solidification of melts obtained from reaction of the free base and the acid.

Crystalline salts may be prepared by directly crystallising from a solvent in which the product has limited solubility, or by triturating or otherwise crystallising a non-crystalline salt. For example, the succinate or the napadisylate may be recrystallised from a variety of organic solvents, such as acetonitrile, butanone, *sec*-butanol, dichloromethane, ethanol, 3-pentanone, propan-2-ol and toluene. An improved yield of the salt is obtained by evaporation of some or all of the solvent or by crystallisation at elevated temperature followed by controlled cooling, for example in stages. Careful control of precipitation temperature and seeding may be used to improve the reproducibility of the production process and the particle size distribution and form of the product. In one embodiment, individual polymorphs are crystallized directly from a solution of the salt, although recrystallizing a solution of one polymorph using seeds of another polymorph may also be carried out.

2-(methyloxy)-*N*-[2-methyl-1-phenyl-2-(1-pyrrolidinyl)propyl]-4,6-bis(trifluoromethyl)benzamide may be prepared by the processes set forth below. All forms of succinic acid and napadisylic acid are commercially available.

2-(methyloxy)-*N*-[2-methyl-1-phenyl-2-(1-pyrrolidinyl)propyl]-4,6-bis(trifluoromethyl)benzamide succinate or the napadisylate may be obtained as a solvate, when during isolation from solution it becomes associated with the solvent in which it is dissolved. Any such solvate forms a further aspect of this invention. Solvates may be returned to the unsolvated succinate or the napadisylate salt by heating, for example by heating a hydrate form above 70°C.

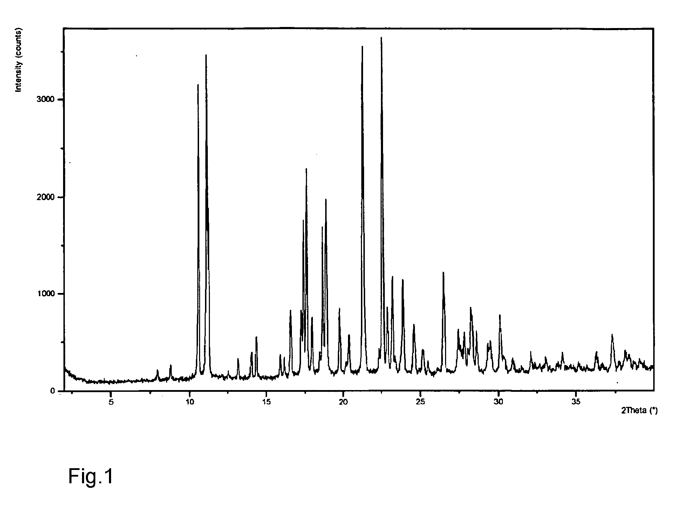

The present invention also provides 2-(methyloxy)-*N*-[2-methyl-1-phenyl-2-(1-pyrrolidinyl)propyl]-4,6-bis(trifluoromethyl)benzamide succinate **characterised in that** it provides an XRPD diffractogram substantially as illustrated in Figure 1.

The present invention further provides 2-(methyloxy)-*N*-[2-methyl-1-phenyl-2-(1-pyrrolidinyl)propyl]-4,6-bis(trifluoromethyl)benzamide succinate **characterised in that** it provides an XRPD diffractogram with signals substantially as listed in Table 1.

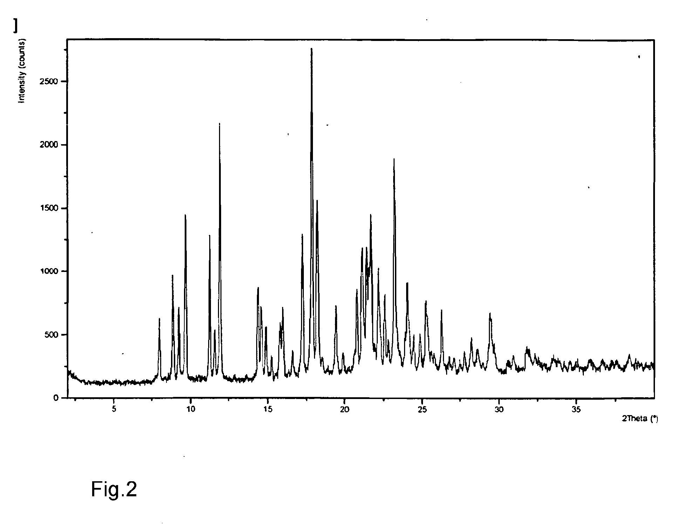

The present invention also provides 2-(methyloxy)-*N*-[2-methyl-1-phenyl-2-(1-pyrrolidinyl)propyl]-4,6-bis(trifluoromethyl)benzamide napadisylate, **characterised in that** it provides an XRPD diffractogram substantially as illustrated in Figure 2.

The present invention further provides 2-(methyloxy)-*N*-[2-methyl-1-phenyl-2-(1-pyrrolidinyl)propyl]-4,6-bis(trifluoromethyl)benzamide napadisylate, **characterised in that** it provides an XRPD diffractogram with signals substantially as listed in Table 2.

In one particular aspect of the invention, the peaks of 2-(methyloxy)-*N*-[2-methyl-1-phenyl-2-(1-pyrrolidinyl)propyl]-4,6-bis(trifluoromethyl)benzamide succinate occur at the following positions, expressed in 2 theta angles (±0.1 degrees): 10.6, 11.1, 21.3 degrees.

In one embodiment, there is provided 2-(methyloxy)-*N*-[2-methyl-1-phenyl-2-(1-pyrrolidinyl)propyl]-4,6-bis(trifluoromethyl)benzamide succinate, **characterised in that** it provides an X-ray powder diffraction pattern comprising 2 theta angles at one or more positions selected from the group consisting of 10.6±0.1, 11.1±0.1, 21.3±0.1 degrees. In another embodiment, there is provided 2-(methyloxy)-*N*-[2-methyl-1-phenyl-2-(1-pyrrolidinyl)propyl]-4,6-bis(trifluoromethyl)benzamide succinate, **characterised in that** it provides an X-ray powder diffraction pattern comprising 2 theta angles at two or more positions selected from the group consisting of 10.6±0.1, 11.1±0.1, 21.3±0.1 degrees.

2-(methyloxy)-*N*-[2-methyl-1-phenyl-2-(1-pyrrolidinyl)propyl]-4,6-bis(trifluoromethyl)benzamide has been found to be an inhibitor of the glycine transporter type GlyT1. Compounds which are inhibitors of GlyT1 are useful in the treatment of disease states which require modulation of GlyT1. 2-(methyloxy)-*N*-[2-methyl-1-phenyl-2-(1-pyrrolidinyl)propyl]-4,6-bis(trifluoromethyl)benzamide has also been found to have greater affinity for GlyT1 than for GlyT2.

Thus the compounds are suitable for the treatment of certain neurological and neuropsychiatric disorders. As used herein, the terms "treatment" and "treating" refer to the alleviation and/or cure of established symptoms as well as prophylaxis. As used herein, the term "psychotic disorder" includes the term "psychiatric disorder".

Therefore, the invention provides 2-(methyloxy)-*N*-[2-methyl-1-phenyl-2-(1-pyrrolidinyl)propyl]-4,6-bis(trifluoromethyl)benzamide succinate or a solvate thereof or 2-(methyloxy)-*N*-[2-methyl-1-phenyl-2-(1-pyrrolidinyl)propyl]-4,6-bis(trifluoromethyl)benzamide napadisylate or a solvate thereof for use in therapy.

In particular the invention provides 2-(methyloxy)-*N*-[2-methyl-1-phenyl-2-(1-pyrrolidinyl)propyl]-4,6-bis(trifluoromethyl)benzamide succinate or a solvate thereof or 2-(methyloxy)-*N*-[2-methyl-1-phenyl-2-(1-pyrrolidinyl)propyl]-4,6-bis(trifluoromethyl)benzamide napadisylate or a solvate thereof for use in the treatment of a disorder mediated by GlyT1.

As used herein, the term "a disorder mediated by GlyT1" refers to a disorder that may be treated by the administration of a medicament that alters the activity of the GlyT1 transporter. As hereinbefore described, the action of GlyT1 transporters affects the local concentration of glycine around NMDA receptors. As a certain amount of glycine is needed for the efficient functioning of NMDA receptors, any change to that local concentration can affect NMDA-mediated neurotransmission. As hereinbefore described, changes in NMDA-mediated neurotransmission have been implicated in certain neuropsychiatric disorders such as dementia, depression and psychoses, for example schizophrenia, and learning and memory disorders, for example attention deficit disorders and autism. Thus, alterations in the activity of the GlyT1 transporter are expected to influence such disorders.

The disorders mediated by GlyT1 referred to herein include neurological and neuropsychiatric disorders, including psychoses such as schizophrenia, dementia and other forms of impaired cognition such as attention deficit disorders and organic brain syndromes. Other neuropsychiatric disorders include drug-induced (phencyclidine, ketamine and other dissociative anesthetics, amphetamine and other psychostimulants and cocaine) psychosis, psychosis associated with affective disorders, brief reactive psychosis, schizoaffective psychosis, and psychosis NOS, "schizophrenia-spectrum" disorders such as schizoid or schizotypal personality disorders, or illness associated with psychosis (such as major depression, manic depressive (bipolar) disorder, Alzheimer's disease and post-traumatic stress syndrome), and disorders such as autism, depression, benign forgetfulness, childhood learning disorders, closed head injury, emesis, aggression, gastric mobility disorders and vertigo.

Within the context of the present invention, the terms used herein are classified in the Diagnostic and Statistical Manual of Mental Disorders, 4th Edition, published by the American Psychiatric Association (DSM-IV) and/or the International Classification of Diseases, 10^{th} Edition (ICD-10). The various subtypes of the disorders mentioned herein are contemplated as part of the present invention. Numbers in brackets after the listed diseases below refer to the classification code in DSM-IV.

2-(methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate are of use in the treatment of schizophrenia including the subtypes Paranoid Type (295.30), Disorganised Type (295.10), Catatonic Type (295.20), Undifferentiated Type (295.90) and Residual Type (295.60); Schizophreniform Disorder (295.40); Schizoaffective Disorder (295.70) including the subtypes Bipolar Type and Depressive Type; Delusional Disorder (297.1) including the subtypes Erotomanic Type, Grandiose Type, Jealous Type, Persecutory Type, Somatic Type, Mixed Type and Unspecified Type; Brief Psychotic Disorder (298.8); Shared Psychotic Disorder (297.3); Psychotic Disorder Due to a General Medical Condition including the subtypes With Delusions and With Hallucinations; Substance-Induced Psychotic Disorder including the subtypes With Delusions (293.81) and With Hallucinations (293.82); and Psychotic Disorder Not Otherwise Specified (298.9).

2-(Methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate are also of use in the treatment of mood disorders including Major Depressive Episode, Manic Episode, Mixed Episode and Hypomanic Episode; Depressive Disorders including Major Depressive Disorder, Dysthymic Disorder (300.4), Depressive Disorder Not Otherwise Specified (311); Bipolar Disorders including Bipolar I Disorder, Bipolar II Disorder (Recurrent Major Depressive Episodes with Hypomanic Episodes) (296.89), Cyclothymic Disorder (301.13) and Bipolar Disorder Not Otherwise Specified (296.80); Other Mood Disorders including Mood Disorder Due to a General Medical Condition (293.83) which includes the subtypes With Depressive Features, With Major Depressive-like Episode, With Manic Features and With Mixed Features), Substance-Induced Mood Disorder (including the subtypes With Depressive Features, With Manic Features and With Mixed Features) and Mood Disorder Not Otherwise Specified (296.90).

2-(Methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate are also of use in the treatment of anxiety disorders including Panic Attack, Agoraphobia, Panic Disorder, Agoraphobia Without History of Panic Disorder (300.22), Specific Phobia (300.29) including the subtypes Animal Type, Natural Environment Type, Blood-Injection-Injury Type, Situational Type and Other Type), Social Phobia (300.23), Obsessive-Compulsive Disorder (300.3), Posttraumatic Stress Disorder (309.81), Acute Stress Disorder (308.3), Generalized Anxiety Disorder (300.02), Anxiety Disorder Due to a General Medical Condition (293.84), Substance-Induced Anxiety Disorder and Anxiety Disorder Not Otherwise Specified (300.00).

2-(Methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate are also of use in the treatment of substance-related disorders including Substance Use Disorders such as Substance Dependence and Substance Abuse; Substance-Induced Disorders such as Substance Intoxication, Substance Withdrawal, Substance-Induced Delirium, Substance-Induced Persisting Dementia, Substance-Induced Persisting Amnestic Disorder, Substance-Induced Psychotic Disorder, Substance-Induced Mood Disorder, Substance-Induced Anxiety Disorder, Substance-Induced Sexual Dysfunction, Substance-Induced Sleep Disorder and Hallucinogen Persisting Perception Disorder (Flashbacks); Alcohol-Related Disorders such as Alcohol Dependence (303.90), Alcohol Abuse (305.00), Alcohol Intoxication (303.00), Alcohol Withdrawal (291.81), Alcohol Intoxication Delirium, Alcohol Withdrawal Delirium, Alcohol-Induced Persisting Dementia, Alcohol-Induced Persisting Amnestic Disorder, Alcohol-Induced Psychotic Disorder, Alcohol-Induced Mood Disorder, Alcohol-Induced Anxiety Disorder, Alcohol-Induced Sexual Dysfunction, Alcohol-Induced Sleep Disorder and Alcohol-Related Disorder Not Otherwise Specified (291.9); Amphetamine (or Amphetamine-Like)-Related Disorders such as Amphetamine Dependence (304.40), Amphetamine Abuse (305.70), Amphetamine Intoxication (292.89), Amphetamine Withdrawal (292.0), Amphetamine Intoxication Delirium, Amphetamine Induced Psychotic Disorder, Amphetamine-Induced Mood Disorder, Amphetamine-Induced Anxiety Disorder, Amphetamine-Induced Sexual Dysfunction, Amphetamine-Induced Sleep Disorder and Amphetamine-Related Disorder Not Otherwise Specified (292.9); Caffeine Related Disorders such as Caffeine Intoxication (305.90), Caffeine-Induced Anxiety Disorder, Caffeine-Induced Sleep Disorder and Caffeine-Related Disorder Not Otherwise Specified (292.9); Cannabis-Related Disorders such as Cannabis Dependence (304.30), Cannabis Abuse (305.20), Cannabis Intoxication (292.89), Cannabis Intoxication Delirium, Cannabis-Induced Psychotic Disorder, Cannabis-Induced Anxiety Disorder and Cannabis-Related Disorder Not Otherwise Specified (292.9); Cocaine-Related Disorders such as Cocaine Dependence (304.20), Cocaine Abuse (305.60), Cocaine Intoxication (292.89), Cocaine Withdrawal (292.0), Cocaine Intoxication Delirium, Cocaine-Induced Psychotic Disorder, Cocaine-Induced Mood Disorder, Cocaine-Induced Anxiety Disorder, Cocaine-Induced Sexual Dysfunction, Cocaine-Induced Sleep Disorder and Cocaine-Related Disorder Not Otherwise Specified (292.9); Hallucinogen-Related Disorders such as Hallucinogen Dependence (304.50), Hallucinogen Abuse (305.30), Hallucinogen Intoxication (292.89), Hallucinogen Persisting Perception Disorder (Flashbacks) (292.89), Hallucinogen Intoxication Delirium, Hallucinogen-Induced Psychotic Disorder, Hallucinogen-Induced Mood Disorder, Hallucinogen-Induced Anxiety Disorder and Hallucinogen-Related Disorder Not Otherwise Specified (292.9); Inhalant-Related Disorders such as Inhalant Dependence (304.60), Inhalant Abuse (305.90), Inhalant Intoxication (292.89), Inhalant Intoxication Delirium, Inhalant-Induced Persisting Dementia, Inhalant-Induced Psychotic Disorder, Inhalant-Induced Mood Disorder, Inhalant-Induced Anxiety Disorder and Inhalant-Related Disorder Not Otherwise Specified (292.9); Nicotine-Related Disorders such as Nicotine Dependence (305.1), Nicotine Withdrawal (292.0) and Nicotine-Related Disorder Not Otherwise Specified (292.9); Opioid-Related Disorders such as Opioid Dependence (304.00), Opioid Abuse (305.50), Opioid Intoxication (292.89), Opioid Withdrawal (292.0), Opioid Intoxication Delirium, Opioid-Induced Psychotic Disorder, Opioid-Induced Mood Disorder, Opioid-Induced Sexual Dysfunction, Opioid-Induced Sleep Disorder and Opioid-Related Disorder Not Otherwise Specified (292.9); Phencyclidine (or Phencyclidine-Like)-Related Disorders such as Phencyclidine Dependence (304.60), Phencyclidine Abuse (305.90), Phencyclidine Intoxication (292.89), Phencyclidine Intoxication Delirium, Phencyclidine-Induced Psychotic Disorder, Phencyclidine-Induced Mood Disorder, Phencyclidine-Induced Anxiety Disorder and Phencyclidine-Related Disorder Not Otherwise Specified (292.9); Sedative-, Hypnotic-, or Anxiolytic-Related Disorders such as Sedative, Hypnotic, or Anxiolytic Dependence (304.10), Sedative, Hypnotic, or Anxiolytic Abuse (305.40), Sedative, Hypnotic, or Anxiolytic Intoxication (292.89), Sedative, Hypnotic, or Anxiolytic Withdrawal (292.0), Sedative, Hypnotic, or Anxiolytic Intoxication Delirium, Sedative, Hypnotic, or Anxiolytic Withdrawal Delirium, Sedative-, Hypnotic-, or Anxiolytic-Persisting Dementia, Sedative-, Hypnotic-, or Anxiolytic- Persisting Amnestic Disorder, Sedative-, Hypnotic-, or Anxiolytic-Induced Psychotic Disorder, Sedative-, Hypnotic-, or Anxiolytic-Induced Mood Disorder, Sedative-, Hypnotic-, or Anxiolytic-Induced Anxiety Disorder Sedative-, Hypnotic-, or Anxiolytic-Induced Sexual Dysfunction, Sedative-, Hypnotic-, or Anxiolytic-Induced Sleep Disorder and Sedative-, Hypnotic-, or Anxiolytic-Related Disorder Not Otherwise Specified (292.9); Polysubstance-Related Disorder such as Polysubstance Dependence (304.80); and Other (or Unknown) Substance-Related Disorders such as Anabolic Steroids, Nitrate Inhalants and Nitrous Oxide.

2-(methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate are also of use in the treatment of sleep disorders including primary sleep disorders such as Dyssomnias such as Primary Insomnia (307.42), Primary Hypersomnia (307.44), Narcolepsy (347), Breathing-Related Sleep Disorders (780.59), Circadian Rhythm Sleep Disorder (307.45) and Dyssomnia Not Otherwise Specified (307.47); primary sleep disorders such as Parasomnias such as Nightmare Disorder (307.47), Sleep Terror Disorder (307.46), Sleepwalking Disorder (307.46) and Parasomnia Not Otherwise Specified (307.47); Sleep Disorders Related to Another Mental Disorder such as Insomnia Related to Another Mental Disorder (307.42) and Hypersomnia Related to Another Mental Disorder (307.44); Sleep Disorder Due to a General Medical Condition; and Substance-Induced Sleep Disorder including the subtypes Insomnia Type, Hypersomnia Type, Parasomnia Type and Mixed Type.

2-(methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate are also of use in the treatment of eating disorders such as Anorexia Nervosa (307.1) including the subtypes Restricting Type and Binge-Eating/Purging Type; Bulimia Nervosa (307.51) including the subtypes Purging Type and Nonpurging Type; Obesity; Compulsive Eating Disorder; and Eating Disorder Not Otherwise Specified (307.50).

2-(methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate are also of use in the treatment of Autistic Disorder (299.00); Attention-Deficit /Hyperactivity Disorder including the subtypes Attention-Deficit /Hyperactivity Disorder Combined Type (314.01), Attention-Deficit /Hyperactivity Disorder Predominantly Inattentive Type (314.00), Attention-Deficit /Hyperactivity Disorder Hyperactive-Impulse Type (314.01) and Attention-Deficit /Hyperactivity Disorder Not Otherwise Specified (314.9); Hyperkinetic Disorder; Disruptive Behaviour Disorders such as Conduct Disorder including the subtypes childhood-onset type (321.81), Adolescent-Onset Type (312.82) and Unspecified Onset (312.89), Oppositional Defiant Disorder (313.81) and Disruptive Behaviour Disorder Not Otherwise Specified; and Tic Disorders such as Tourette's Disorder (307.23).

2-(methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-I-phenyl-2-(1-pyrrolidinyl)propyl]-4,6-bis(trifluoromethyl)benzamide napadisylate are also of use in the treatment of Personality Disorders including the subtypes Paranoid Personality Disorder (301.0), Schizoid Personality Disorder (301.20), Schizotypal Personality Disorder (301,22), Antisocial Personality Disorder (301.7), Borderline Personality Disorder (301,83), Histrionic Personality Disorder (301.50), Narcissistic Personality Disorder (301,81), Avoidant Personality Disorder (301.82), Dependent Personality Disorder (301.6), Obsessive-Compulsive Personality Disorder (301.4) and Personality Disorder Not Otherwise Specified (301.9).

2-(methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate are also of use in the enhancement of cognition including the treatment of cognition impairment in other diseases such as schizophrenia, bipolar disorder, depression, other psychotic disorders and psychotic conditions associated with cognitive impairment. Within the context of the present invention, the term cognitive impairment includes for example the treatment of impairment of cognitive functions induing attention, orientation, learning disorders, memory (i.e. memory disorders, amnesia, amnesic disorders, transient global amnesia syndrome and age-associated memory impairment) and language function; cognitive impairment as a result of stroke, Alzheimer's disease, Huntington's disease, Pick disease, Aids-related dementia or other dementia states such as Multiinfarct dementia, alcoholic dementia, hypotiroidism-related dementia, and dementia associated to other degenerative disorders such as cerebellar atrophy and amyotropic lateral sclerosis; other acute or sub-acute conditions that may cause cognitive decline such as delirium or depression (pseudodementia states) trauma, head trauma, age related cognitive decline, stroke, neurodegeneration, drug-induced states, neurotoxic agents, mild cognitive impairment, age related cognitive impairment, autism related cognitive impairment, Down's syndrome, cognitive deficit related to psychosis, and post-electroconvulsive treatment related cognitive disorders; and dyskinetic disorders such as Parkinson's disease, neuroleptic-induced parkinsonism, and tardive dyskinesias.

2-(methyloxy)-*N*-[2-methyl-1-phenyl-2- (1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate are also of use in the treatment of sexual dysfunctions including Sexual Desire Disorders such as Hypoactive Sexual Desire Disorder (302.71), and Sexual Aversion Disorder (302.79); sexual arousal disorders such as Female Sexual Arousal Disorder (302.72) and Male Erectile Disorder (302.72); orgasmic disorders such as Female Orgasmic Disorder (302.73), Male Orgasmic Disorder (302.74) and Premature Ejaculation (302.75); sexual pain disorder such as Dyspareunia (302.76) and Vaginismus (306.51); Sexual Dysfunction Not Otherwise Specified (302.70); paraphilias such as Exhibitionism (302.4), Fetishism (302.81), Frotteurism (302.89), Pedophilia (302.2), Sexual Masochism (302.83), Sexual Sadism (302.84), Transvestic Fetishism (302.3), Voyeurism (302.82) and Paraphilia Not Otherwise Specified (302.9); gender identity disorders such as Gender Identity Disorder in Children (302.6) and Gender Identity Disorder in Adolescents or Adults (302.85); and Sexual Disorder Not Otherwise Specified (302.9).

2-(methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate are also of use as anticonvulsants. 2-(methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate are thus useful in the treatment of convulsions in mammals, and particularly epilepsy in humans. "Epilepsy" is intended to include the following seizures: simple partial seizures, complex partial seizures, secondary generalised seizures, generalised seizures including absence seizures, myoclonic seizures, clonic seizures, tonic seizures, tonic clonic seizures and atonic seizures. 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof is used in the treatment of convulsions.

2-(methyloxy)-*N*-[2-methyl-1-phenyl-2-(1-pyrrolidinyl)propyl]-4,6-bis(trifluoromethyl)benzamide succinate and 2-(methyloxy)-*N*-[2-methyl-1-phenyl-2-(1-pyrrolidinyl)propyl]-4,6-bis(trifluoromethyl)benzamide napadisylate also find use in the treatment of neuropathic pain, for example in diabetic neuropathy, sciatica, non-specific lower back pain, multiple sclerosis pain, fibromyalgia, HIV-related neuropathy, neuralgia such as post-herpetic neuralgia and trigeminal neuralgia and pain resulting from physical trauma, amputation, cancer, toxins or chronic inflammatory conditions.

In another aspect of the invention, there is provided use of 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, in the preparation of a medicament for the treatment of a disorder mediated by GlyT1.

In one embodiment, the disorder mediated by GlyT1 to be treated by the use as hereinbefore described is a psychosis, including schizophrenia, dementia and attention deficit disorders, particularly schizophrenia.

As used herein, the term "effective amount" means that amount of a drug or pharmaceutical agent that will elicit the biological or medical response of a tissue, system, animal or human that is being sought, for instance, by a researcher or clinician.

Compounds for use according to the invention may be administered as the raw material, but in one embodiment, the active ingredients are provided in the form of pharmaceutical compositions.

Accordingly, in a further aspect of the invention, there is provided a pharmaceutical composition comprising 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy-*N*-[2-methyl-1-phenyl-2-(1-pyrrolidinyl)propyl]-4,6-bis(trifluoromethyl)benzamide napadisylate or a solvate thereof, and at least one pharmaceutically acceptable carrier, diluent or excipient.

These pharmaceutical compositions may be used in the treatment of clinical conditions for which a GlyT1 inhibitor is indicated such as, for example, schizophrenia. The carrier must be pharmaceutically acceptable to the recipient and must be compatible with, i.e. not have a deleterious effect upon, the other ingredients in the composition. The carrier may be a solid or a liquid and may be formulated with at least one of 2-(methyloxy)-*N*-[2-methyl-1-phenyl-2-(1-pyrrolidinyl)propyl]-4,6-bis(trifluoromethyl)benzamide succinate or a solvate thereof, and 2-(methyloxy)-*N*-[2-methyl-1-phenyl-2-(1-pyrrolidinyl)propyl]-4,6-bis(trifluoromethyl)benzamide napadisylate or a solvate thereof, as a unit dose formulation. If desired, other active ingredients may also be incorporated in the pharmaceutical compositions of the invention.

It will be appreciated by those skilled in the art that the compounds according to the invention may advantageously be used in conjunction with one or more other therapeutic agents, for instance, different antidepressant agents such as 5HT3 antagonists, serotonin agonists, NK-1 antagonists, selective serotonin reuptake inhibitors (SSRI), noradrenaline reuptake inhibitors (SNRI), tricyclic antidepressants, dopaminergic antidepressants, H3 antagonists, 5HT1A antagonists, 5HT1B antagonists, 5HT1D antagonists, D1 agonists, M1 agonists and/or anticonvulsant agents, as well as typical and atypical antipsychotic drugs and cognitive enhancers.

Suitable 5HT3 antagonists which may be used in combination of the compounds of the inventions include for example ondansetron, granisetron, metoclopramide.

Suitable serotonin agonists which may be used in combination with the compounds of the invention include sumatriptan, rauwolscine, yohimbine, metoclopramide.

Suitable SSRIs which may be used in combination with the compounds of the invention include fluoxetine, citalopram, femoxetine, fluvoxamine, paroxetine, indalpine, sertraline, zimeldine.

Suitable SNRIs which may be used in combination with the compounds of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

Suitable anticonvulsant agents which may be used in combination of the compounds of the invention include for example divalproex, carbamazepine and diazepam.

Examples of neuroleptic/antipsychotic drugs that are useful in the present invention include, but are not limited to: butyrophenones, such as haloperidol, pimozide, and droperidol; phenothiazines, such as chlorpromazine, thioridazine, mesoridazine, trifluoperazine, perphenazine, fluphenazine, thiflupromazine, prochlorperazine, and acetophenazine; thioxanthenes, such as thiothixene and chlorprothixene ; thienobenzodiazepines; dibenzodiazepines; benzisoxazoles; dibenzothiazepines; imidazolidinones ; benzisothiazolyl-piperazines; triazine such as lamotrigine; dibenzoxazepines, such as loxapine; dihydroindolones, such as molindone; aripiprazole; and derivatives thereof that have antipsychotic activity.

Examples of tradenames and suppliers of selected neuroleptic drugs are as follows : clozapine (available under the tradename CLOZARIL®, from Mylan, Zenith Goldline, UDL, Novartis); olanzapine (available under the tradename ZYPREX®, from Lilly ; ziprasidone (available under the tradename GEODON®, from Pfizer); risperidone (available under the tradename RISPERDAL®, from Janssen); quetiapine fumarate (available under the tradename SEROQUEL®, from AstraZeneca); haloperidol (available under the tradename HALDOL®, from Ortho-McNeil); chlorpromazine (available under the tradename THORAZINE®, from SmithKline Beecham (GSK); fluphenazine (available under the tradename PROLIXIN®, from Apothecon, Copley, Schering, Teva, and American Pharmaceutical Partners, Pasadena); thiothixene (available under the tradename NAVANE®;, from Pfizer); trifluoperazine (10-[3-(4-methyl-1-piperazinyl)propyl]-2-(trifluoromethyl)phenothiazine dihydrochloride, available under the tradename STELAZINE®, from Smith Klein Beckman; perphenazine (available under the tradename TRILAFON®; from Schering); thioridazine (available under the tradename MELLARIL®; from Novartis, Roxane, HiTech, Teva, and Alpharma) ; molindone (available under the tradename MOBAN®, from Endo); and loxapine (available under the tradename LOXITANE®; from Watson). Furthermore, benperidol (Glianimon®), perazine (Taxilan®) or melperone (Eunerpan®)) may be used. Other neuroleptic drugs include promazine (available under the tradename SPARINE®), triflurpromazine (available under the tradename VESPRIN®), chlorprothixene (available under the tradename TARACTAN®), droperidol (available under the tradename INAPSINE®), acetophenazine (available under the tradename TINDAL®;), prochlorperazine (available under the tradename COMPAZINE®), methotrimeprazine (available under the tradename NOZINAN®), pipotiazine (available under the tradename PIPOTRIL®), ziprasidone, and hoperidone.

In one embodiment, the neuroleptic agent for use in the invention is olanzapine, risperidone, quetiapine, aripiprazole, haloperidol, clozapine, ziprasidone or osanetant.

It will be appreciated that the compounds of the combination or composition may be administered simultaneously (either in the same or different pharmaceutical formulations), separately or sequentially.

In one embodiment, the combination therapies of the invention are administered adjunctively. By adjunctive administration is meant the coterminous or overlapping administration of each of the components in the form of separate pharmaceutical compositions or devices. This regime of therapeutic administration of two or more therapeutic agents is referred to generally by those skilled in the art and herein as adjunctive therapeutic administration; it is also known as add-on therapeutic administration.

Any and all treatment regimes in which a patient receives separate but coterminous or overlapping therapeutic administration of the compounds of the present invention or a pharmaceutically acceptable salt or solvate thereof and at least one neuroleptic agent are within the scope of the current invention. In one embodiment of adjunctive therapeutic administration as described herein, a patient is typically stabilised on a therapeutic administration of one or more of the components for a period of time and then receives administration of another component. Within the scope of this invention, 2-(methyloxy)-*N-*[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, may be administered as adjunctive therapeutic treatment to patients who are receiving administration of at least one neuroleptic agent, but the scope of the invention also includes the adjunctive therapeutic administration of at least one neuroleptic agent to patients who are receiving administration 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof.

The combination therapies of the invention may also be administered simultaneously. By simultaneous administration is meant a treatment regime wherein the individual components are administered together, either in the form of a single pharmaceutical composition or device comprising or containing both components, or as separate compositions or devices, each comprising one of the components, administered simultaneously. Such combinations of the separate individual components for simultaneous combination may be provided in the form of a kit-of-parts.

In a further aspect, the invention provides the use of 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, in the manufacture of a medicament for adjunctive therapeutic administration for the treatment of a psychotic disorder in a patient receiving therapeutic administration of at least one neuroleptic agent. The invention further provides 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N-*[2-methyl-1-phenyl-2-(1-pyrrolidinyl)propyl]-4,6-bis(trifluoromethyl)benzamide napadisylate or a solvate thereof, for use for adjunctive therapeutic administration for the treatment of a psychotic disorder in a patient receiving therapeutic administration of at least one neuroleptic agent.

In a further aspect, the invention provides the use of at least one neuroleptic agent in the manufacture of a medicament for adjunctive therapeutic administration for the treatment of a psychotic disorder in a patient receiving therapeutic administration of 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof. The invention further provides at least one neuroleptic agent for adjunctive therapeutic administration for the treatment of a psychotic disorder in a patient receiving therapeutic administration of 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyt-2-(1-pyrrolidinyt)propyl]-4,6-bis(trifluoromethyl)benzamide napadisylate or a solvate thereof.

The invention further provides the use of a combination of 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, and at least one neuroleptic agent in the manufacture of a medicament for simultaneous therapeutic administration in the treatment of a psychotic disorder. The invention further provides the use of 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N-*[2-methyl-1-phenyl-2-(1-pyrrolidinyl)propyl]-4,6-bis(trifluoromethyl)benzamide napadisylate or a solvate thereof, in the manufacture of a medicament for simultaneous therapeutic administration with at least one neuroleptic agent in the treatment of a psychotic disorder. The invention further provides 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, for use for simultaneous therapeutic administration with at least one neuroleptic agent in the treatment of a psychotic disorder. The invention further provides the use of at least one neuroleptic agent in the manufacture of a medicament for simultaneous therapeutic administration with compounds 2-(methyloxy)-*N*-[2-methyl-1-phenyl-2-(1-pyrrolidinyl)propyl]-4,6-bis(trifluoromethyl)benzamide succinate or a solvate thereof, or 21-(methyloxy)-*N*-[2-methyl-1-phenyl-2-(1-pyrrolidinyl)propyl]-4,6-bis(trifluoromethyl)benzamide napadisylate or a solvate thereof, in the treatment of a psychotic disorder.

In further aspects, the invention provides a medicament for a method of treatment of a psychotic disorder by simultaneous therapeutic administration of a pharmaceutical composition comprising 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, and at least one mood stabilising or antimanic agent, a pharmaceutical composition comprising 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, and at least one mood stabilising or antimanic agent, the use of a pharmaceutical composition comprising 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, and at least one mood stabilising or antimanic agent in the manufacture of a medicament for the treatment of a psychotic disorder, and a pharmaceutical composition comprising 2-(methyloxy)-*N*-[2-methyl-1-phenyl-2-(1-pyrrolidinyi)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, and at least one mood stabilising or antimanic agent for use in the treatment of a psychotic disorder.

In a further aspect, the invention provides a kit-of-parts for use in the treatment of a psychotic disorder comprising a first dosage form 2-(methyloxy)-*N*-[2-methyl-1-phenyl-2-(1-pyrrolidinyl)propyl]-4,6-bis(trifluoromethyl)benzamide succinate or a solvate thereof, or 2-(methyloxy)-*N*-[2-methyl-1-phenyl-2-(1-pyrrolidinyl)propyl]-4,6-bis(trifluoromethyl)benzamide napadisylate or a solvate thereof, and one or more further dosage forms each comprising a neuroleptic agent for simultaneous therapeutic administration.

Within the context of the present invention, the term psychotic disorder includes those disorders mentioned above, such as schizophrenia, mood disorders, anxiety disorders, substance-related disorders, sleep disorders, eating disorders, autistic disorder, attention-deficit/hyperactivity disorder, disruptive behaviour disorder, tic disorders, personality disorders, cognition impairment in other diseases, sexual dysfunction, dyskinetic disorders, depression, bipolar disorder, cognitive impairment and obsessive-compulsive disorders and all the various forms of the disorders as mentioned herein. which are contemplated as part of the present invention.

For use in medicine, the compounds of the present invention are usually administered as a standard pharmaceutical composition. The present invention therefore provides in a further aspect a pharmaceutical composition comprising 2-(methyloxyry)-*N*-[2-methyl-1-phenyl-2-(1-pyrrolidinyl)propyl]-4,6-bis(trifluoromethyl)benzamide succinate or a solvate thereof and a pharmaceutically acceptable carrier. The present invention also provides 2-(methyloxy)-*N-*[2-methyl-1-phenyl-2-(1-pyrrolidinyl)propyl]-4,6-bis(trifluoromethyl)benzamide napadisylate or a solvate thereof and a pharmaceutically acceptable carrier. The pharmaceutical composition can be for use in the treatment of any of the conditions described herein.

Possible formulations include those suitable for oral, sub-lingual, buccal, parenteral (for example, subcutaneous, intramuscular, or intravenous), rectal, topical and intranasal administration and in forms suitable for administration by inhalation or insufflation (either through the mouth or nose). The most suitable means of administration for a particular patient will depend on the nature and severity of the conditions being treated and on the nature of the active compound. In one embodiment, oral administration is provided

Formulations suitable for oral administration may be provided as discrete units, such as tablets, capsules, cachets, or lozenges, each containing a predetermined amount of the active compound; as powders or granules; as solutions or suspensions in aqueous or nonaqueous liquids; or as oil-in-water or water-in-oil emulsions. For example, a compound of the invention may be prepared as a formulation with a controlled release profile. This may be in any of the above mentioned pharmaceutical forms. For example, it may be a gel formulation in a non aqueous oily vehicle, for example Miglyol, with a suitable gelling agent if required, for example methyl cellulose or hydrophobic colloidal silica.

Formulations suitable for sublingual or buccal administration include lozenges comprising the active compound and, typically, a flavoured base, such as sugar and acacia or tragacanth and pastilles comprising the active compound in an inert base, such as gelatin and glycerin or sucrose and acacia.

Formulations suitable for parenteral administration typically comprise sterile aqueous solutions containing a predetermined concentration of the active compound; the solution may be isotonic with the blood of the intended recipient. Although such solutions may be administered intraveneously, they may also be administered by subcutaneous or intramuscular injection.

Formulations suitable for rectal administration may be provided as unit-dose suppositories comprising the active ingredient and one or more solid carriers forming the suppository base, for example, cocoa butter.

Formulations suitable for topical or intranasal application include ointments, creams, lotions, pastes, gels, sprays, aerosols and oils. Suitable carriers for such formulations include petroleum jelly, lanolin, polyethylene glycols, alcohols, and combinations thereof.

Formulations of compounds of the invention may, for example, be composed so as to improve the exposure profile of the compound of the invention.

Compositions suitable for transdermal administration include ointments, gels and patches. In one embodiment, the composition is in unit dose form such as a tablet, capsule or ampoule.

The formulations of the invention may be prepared by any suitable method, typically by uniformly and intimately admixing the active compound(s) with liquids or finely divided solid carriers, or both, in the required proportions and then, if necessary, shaping the resulting mixture into the desired shape.

For example, a tablet may be prepared by compressing an intimate mixture comprising a powder or granules of the active ingredient and one or more optional ingredients, such as a binder, lubricant, inert diluent, or surface active dispersing agent, or by moulding an intimate mixture of powdered active ingredient and inert liquid diluent.

Aqueous solutions for parenteral administration are typically prepared by dissolving the active compound in sufficient water to give the desired concentration and then rendering the resulting solution sterile and isotonic.

It will be appreciated that the precise dose administered will depend on the age and condition of the patient and the frequency and route of administration and will be at the ultimate discretion of the attendant physician. The compound may be administered in single or divided doses and may be administered one or more times, for example 1 to 4 times per day.

A proposed dose of the active ingredient for use according to the invention for oral, sublingual, parenteral, buccal, rectal, intranasal or topical administration to a human (of approximately 70 kg bodyweight) for the treatment of a psychotic disorder mediated by a GlyT1 inhibitor, including schizophrenia, may be about 1 to about 1000 mg, such as about 5 to about 500 mg, or about 10 to about 100 mg of the active ingredient per unit dose which could be administered, for example, 1 to 4 times per day.

Compounds of the invention may be used as PET ligands (for example labelled with carbon-11 or fluorine-18) or as SPECT ligands (for example labelled with iodine-123 or meta stable technetium-99) for *in vivo* visualisation and quantification of the GlyT1 transporter. For example, they may be used in PET or SPECT imaging of the brain. In the context of this patent, PET shall mean: positron emission tomography and SPECT (= SPET) shall mean: single photon emission (computed) tomography.

The invention is further illustrated by the following non-limiting examples.

### Abbreviations:

- THF: tetrahydrofuran
- DCM: dichloromethane
- DMF: dimethylformamide
- HATU: O-(7-azabenzotriazol-1-yl)-N,N,N',N'-tetramethyluronium hexafluorophosphate
- EDC: N-(3-(dimethylamino)propyl)-N-ethylcarbodiimide hydrochloride
- HOAt: 3H-(1,2,3)-triazolo(4,5-b)pyridine-3-ol
- EtOAc: ethyl acetate
- NMP: N-methylpyrrolidinone
- DIPEA: N,N-diisopropylethylamine
- HOBt: 1-hydroxybenzotriazole hydrate
- IPA: 2-propanol
- TBTU: O-benzotriazol-1-yl-N,N,N',N'-tetramethyluronium tetrafluoroborate
- DMSO: dimethylsulfoxide
- HEPES: 4-(2-hydroxyethyl)-1-piperazineethanesulfonic acid
- AcCN: acetonitrile
- TEA: triethylamine
- MeOH: methanol

### Biological Test Methods

The affinities of the compounds of this invention for the GlyT1 transporter were determined by the following assay:

HEK293 cells expressing the Glycine (Type 1) transporter were grown in cell culture medium [DMEM/NUT mix F12 containing 2mM L-Glutamine, 0.8mg/mL G418 and 10% heat inactivated fetal calf serum] at 37°C. and 5% CO₂. Cells grown to 70-80% confluency in T175 flasks were harvested and frozen. For the assay, cells were defrosted and resuspended at 1.32x106 cells/mL in assay buffer [140mM NaCl, 5.4mM KCI, 1.8mM CaCl₂, 0.8mM MgSO₄, 20mM HEPES, 5mM glucose and 5mM alanine, pH 7.4]. Compounds were serially diluted either 3-fold or 4-fold in DMSO from a top concentration of 2.5mM with each compound giving a 11 data point dose-response. 100nL of compound at each concentration was added to the assay plate. An equal volume of Leadseeker™ WGA SPA beads (12.5mg/ml suspended in assay buffer) was added to the cell suspension (1.32 x 106) and 5uL of the cell/bead suspension transferred to each well of a LV384-well white solid bottom plate (3300 cells/well) containing 100nL of test compounds. Substrate (5uL) was added to each well [1:100 dilution of [3H]-glycine stock in assay buffer containing 2.5uM glycine). Final DMSO concentration was 1% v/v. Data was collected using a Perkin Elmer Viewlux^{™}. pIC₅₀ values were determined using ActivityBase^{™}.

Compounds having a pIC₅₀ at the GlyT1 transporter of greater than or equal to 5.0 are considered to be active at the GlyT1 transporter. R-(-)-2-(methyloxy)-*N*-[2-methyl-1-phenyl-2-(1-pyrrolidinyl)propyl]-4,6-bis(trifluoromethyl)benzamide monosuccinate was found to have an average pIC₅₀ at the GlyT1 transporter of 7.8 (n=5). R-(-)-2-(methyloxy)-*N*-[2-methyl-1-phenyl-2-(1-pyrrolidinyl)propyl]-4,6-bis(trifluoromethyl)benzamide 1,5-heminapadisylate was found to have a pIC₅₀ at the GlyT1 transporter of 8.3 (n=1).

Throughout the examples section, the following terminology is adopted with regard to chiral compounds: when a mixture of two enantiomers has been prepared, the compound is described as (±). When a single enantiomer (that is to say mixture chirally enriched in one of the enantiomers) has been prepared, it is referred to as "chiral". Individual enantiomers of some materials prepared are identified by virtue of optical rotations and such materials are identified as the (+) or (-) enantiomers.

Where reactions are described as having been carried out in a similar manner to earlier, more completely described reactions, the general reaction conditions used were essentially the same. Work up conditions used were of the types standard in the art, but may have been adapted from one reaction to another. The starting material may not necessarily have been prepared from the batch referred to.

### Description 1: 2-Methyl-2-(1-pyrrolidinyl)propanenitrile

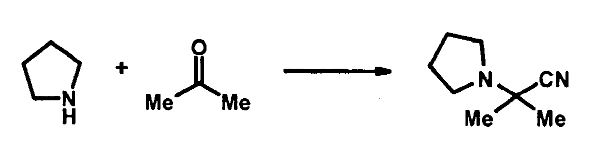

To a stirred, ice-cooled mixture of pyrrolidine (8.35ml; 0.1mol) and acetone (7.34ml; 0.1mol) was added a solution of potassium cyanide (6.51g; 0.1mol) in water (50ml) dropwise over 10min. After stirring at room temperature overnight, the crude reaction mixture was extracted with diethyl ether (2 x 250ml) and the combined extracts washed with saturated brine (150ml), dried (MgSO₄), and evaporated under reduced pressure to afford the title product as a pale green liquid (10.7g; 78%) which was used without further purification. ¹H NMR (CDCl₃) δ: 1.51 (6H, s), 1.80 -1.90 (4H, m), 2.70 - 2.80 (4H, m).

### Description 2: (±)[2-Methyl-1-phenyl-2-(1-pyrrolidinyl)propyl]amine

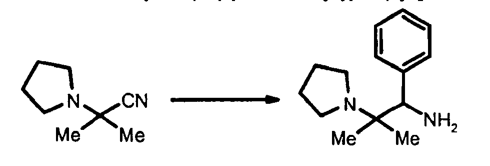

To a solution of 2-methyl-2-(1-pyrrolidinyl)propanenitrile D1 (10.7g; 77.54mmol) in THF (400ml) at -70°C under argon was added over 10 minutes a solution of phenyllithium in dibutylether (86.3ml of a 1.8M solution; 155mmol). The reaction mixture was stirred at - 70°C for 2 hours then allowed to warm to room temperature and stirred overnight. The reaction mixture was cooled in ice as saturated aqueous sodium hydrogen carbonate (400ml) was added. After stirring for a further 30 minutes the layers were separated, and the aqueous layer extracted with ether (200ml). Combined organics were dried (MgSO₄) and evaporated. The residual amber oil was dissolved in methanol (400ml), cooled in ice and sodium borohydride (5.2g; 137mmol) added in four portions over 5 minutes. The reaction mixture was stirred with ice cooling for 30 minutes, the ice removed and stirred at room temperature for 1.5 hours. The mixture was cooled in ice as water (50ml) was added prior to concentration *in vacuo* to approx 70ml. The mixture was partitioned between 2N HCl (100ml) and ethyl acetate (400ml) and the organics extracted with 2N HCl (2 x 100ml). Combined acidic aqueous layers were washed with ethyl acetate (200ml), basified with 50% NaOH and extracted with DCM (3 x 150ml). Combined DCM organic extracts were dried (Na₂SO₄) and evaporated *in vacuo* to afford the title compound as a colourless solid (15g: 88%)¹H NMR (CDCl₃), δ:0.75 (3H, s), 0.99 (3H, s), 1.70 - 1.76 (4H, m), 1.80 (2H, bs), 2.65 - 2.70 (4H, m), 4.08 (1H, s), 7.20 - 7.42 (5H, m).

### Description 3: (+)-[2-Methyl-1-phenyl-2-(1-pyrrolidinyl)propyl]amine

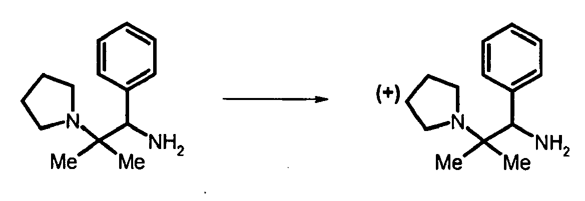

A solution of (R)-(-)-α-methoxyphenylacetic acid (8.08g; 49mmol) in 2-propanol (50ml) was added dropwise over 10 minutes to a stirred solution of [2-methyl-1-phenyl-2-(1-pyrrolidinyl)propyl]amine D2 (10.64g; 49mmol) in 2-propanol (107ml) at 57°C. After complete addition heating was continued for a further 10 minutes. Heating was then removed and stirring continued for one and three quarter hours. Further 2-propanol (100ml)) was added and the mixture filtered and the solid washed with 2-propanol (3 x 50ml), ether (100ml) and dried. The solid was recrystallised from boiling 2-propanol (1L) and the crystals filtered, washed with cold 2-propanol, ether and dried. A sample was partitioned between saturated aqueous sodium hydrogen carbonate and DCM and the organic layer passed through a phase separation cartridge and blown down with argon to afford the title compound as a colourless solid. ¹H NMR (CDCl₃), δ:0.75 (3H, s), 0.99 (3H, s), 1.70 - 1.79 (4H, m), 1.85 (2H, bs), 2.65 - 2.70 (4H, m), 4.08 (1H. s), 7.20 - 7.42 (5H, m). Chiral HPLC: 97.5% ee, corresponding to the slower running enantiomer 2. [α]_{D} = +28.5° (c=1, CHCl₃ at 27.5°C). The remaining free base was liberated in a similar manner (3.55g, 66%).

Conditions for resolution of racemate D2 were as follows:-

| | |
|---|---|
| Column: | chiralcel OD-H 5 um, 250 x 4.6 mm i.d. |
| | 10 micron particle size |
| Mobile phase: | Heptane : Ethanol (90:10) |
| Gradient: | isocratic |
| Flow rate: | 1 ml/min |
| UV wavelength range: | 254 nm |
| Analysis time: | 10 min |
| Ret. Time: | 5.4min (Enantiomer 1); 7.0min (Enantiomer 2) |

### Description D3a: Enantioselective Synthesis of (+)-[2-Methyl-1-phenyl-2-(1-pyrrolidinyl)propyl]amine

For the following enantioselective synthesis of (+)-[2-methyl-1-phenyl-2-(1-pyrrolidinyl)propyl]amine, all the reagents and solvents were purchased from AldrichChimica unless specified. Reactions were followed up with the walkup HPLC (acid method, 8 min or 2 min run was effective), or LC-MS. ¹H-NMR was run on the 400 MHz, preparing a solution of the sample in DMSO-d6 or Acetone-d6.

### Stage 1: One pot procedure toward compound 3 in methanol

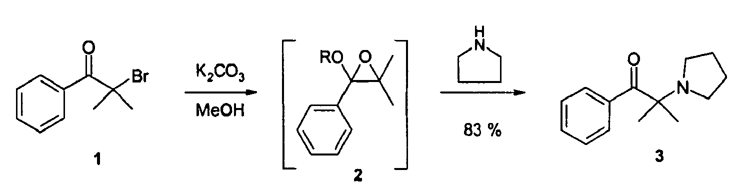

2-Bromoisobutyrophenone **1** (0.05 mol, 11.36 g) and K₂CO₃ 99% (20 g, 2 wt) in methanol (HPLC grade, 25 mL, 2 vol) were stirred at room temperature for 3 hours under nitrogen atmosphere. ¹H-NMR showed completeness. Pyrrolidine 99.5% (10 mL, 0.9 vol) was added to the slurry and heated at 70 °C for 40 hours (HPLC, RT_{RP} 1.00; RT_{SP} 1.31; 2 min method). Workup started although some starting material was left (about 5% a/a). The solution was diluted with TBME (11 mL, 1 vol) and filtered. The residue was rinsed with TBME (10 mL x 2) and the combined organic layer was concentrated at vacuum. The resulting oil was taken up with EtOAc (20 mL, 2 vol) and washed with 2 N HCl (5 mL x 5). The acid solution was brought to pH 12-13 with 6 N aqueous NaOH (about 10 mL, 1 vol) and extracted with EtOAc (8 mL x 3). Evaporation of the organic solution gave **3** as yellow crystals (9.58 g, 0.044 mol, 88 % yield). This reaction was repeated many times, yielding between 75-90 %.

### Stage 1: One pot procedure toward compound 3. Alternative in Ethanol

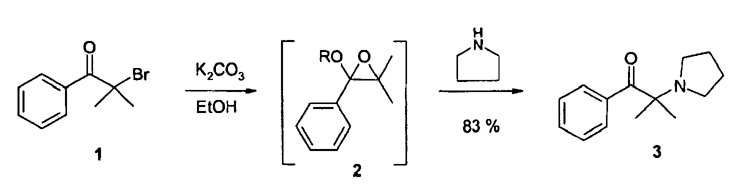

2-Bromoisobutyrophenone **1** (15 mmol, 3.37 g, 2.5 ml) and K₂CO₃ 99% (6 g, 1.8 wt) in ethanol 9 ml, 2.7 vol) were stirred at room temperature overnight under nitrogen atmosphere. Pyrrolidine 99.5% (3.5 ml, 1 vol) was added to the slurry and heated at reflux for 24 hours. The solution was cooled and diluted with EtOAc (20 ml) and filtered. The residue was washed with EtOAc (20 ml). The solvent was washed with water (20 ml x 2) and extracted with 2 N HCl (20 ml x 2). The acid solution was washed with EtOAc (20 ml) then EtOAc (20 ml) was added followed by 6 N aqueous K₂CO₃ to pH 12-13. The EtOAc was separated and the water re-extracted with EtOAc (20 ml). The combined organic solutions were washed with water (20 ml x 2) and concentrated to give the desired product (2.62 g, 81 % yield).

### Stage 2: Synthesis of compound 5

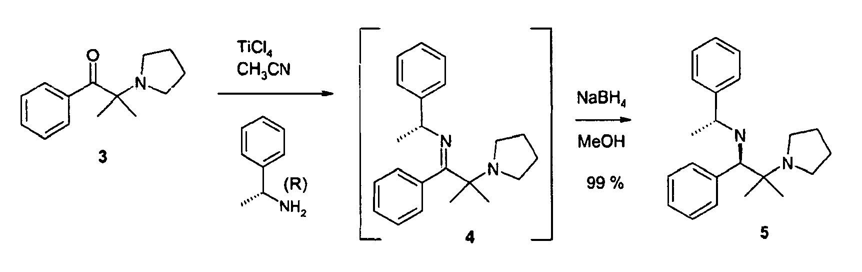

A flask was charged with the pyrrolidineketone **3** (3.90 g, 17.9 mmol, 1 wt), triethylamine 99.5 % (9.36 ml, 4 eq, 0.072 mol) and R-(+)-a-methylbenzylamine (2.60g, 21.5 mmol, 2.77 ml) in acetonitrile (35 ml, 10 vol) under nitrogen atmosphere. 1 M titanium(IV) chloride in dichloromethane (14.3 mL, 3.7 vol) was added dropwise in 15 min, cooling the flask at 5-10 °C (water-ice bath). The resulting slurry was kept at room temperature for 2.5 hours. The mixture was brought to 0 °C and sodium borohydride (1.40 g, 2 eq) was added portionwise followed by dropwise addition of methanol (8 mL, 2 vol). The reaction was slowly brought to room temperature in 2 hours and left overnight with stirring. The slurry was quenched with 4 N HCl (2 ml) and filtered and washed with CH₃CN (2 ml). The solvent was partially evaporated and extracted with EtOAc (8 mL, 2 vol x 2) after addition of 1 N HCl (16 ml, 4 vol). The emulsion was filtered. After filtration 2vol of HCl 2 N and 2 vol of EtOAc were added. The water phase (25ml) was separated and the organic layer was washed with HCl 2N (2 vol, 8 ml). The combined water layer (33ml, 10 vol) was brought to pH 12 with 6 N NaOH and taken up with EtOAc (8 mL x 3). Evaporation gave a yellow-dark oil (5.16 g, 20 mmol, 100%).

### Stage 2: Synthesis of compound 5. Alternative procedure

Pyrrolidineketone **3** (1 g, 4.6 mmol, 1 wt) was dissolved in acetonitrile (8 mL, 9 vol) and R-(+)-a-methylbenzylamine (0.7 mL, 0.7 vol) was added followed by triethylamine 99.5 % (2.5 ml, 2.5 vol) under nitrogen atmosphere. The mixture was stirred at 15-20°C. Then a solution of 1 M titanium(IV) chloride in dichloromethane (4.6 ml) was added dropwise in 15 min at 10 °C (water-ice bath) with vigorous stirring. The funnel was washed with acetonitrile (2 ml, 2 vol). The resulting slurry was stirred at room temperature for 1.5 hours. The mixture was cooled to 0 °C and sodium borohydride (350 mg, 2 eq) was added followed by dropwise addition over 20 min of methanol (4 ml). The reaction was slowly brought to room temperature in 2 hours and left for a further two hours. The solvent was evaporated under vacuum to 5 vol and EtOAc (10 vol) was added. The suspension was filtered on Sterimat and the solid washed with EtOAc (5 vol). The EtOAc was extracted with 2N HCl solution (10 vol x 2) and the combined water solutions were washed with EtOAc (10 vol). EtOAc (10 vol) was added and the combined water layer was brought to pH 12 with solid KOH. The EtOAc was separated and the water re-extracted with EtOAc (10 ml). The organic phases were combined, washed with water (3 x 10 ml) and evaporated to give the desired compound (1.45 g, 97%).

### Stage 3: Synthesis of (+)-[2-methyl-1-phenyl-2-(1-pyrrolidinyl)propyl]amine via direct hydrogenolysis

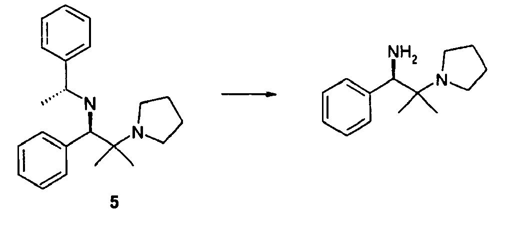

An endeavor tube was charged with the chiral diamine **5** (1.62 g, 1 wt, 5.31 mmol) and dissolved in 10 % conc. sulphuric acid in methanol (HPLC grade, 3 mL, 2 vol), 10 % palladium/carbon (150 mg, 10 % wt; Strem Chemicals, 50 % wet) and submitted for 1 hour at 60°C and 3 atm of hydrogen. The mixture was left to reach room temperature and filtered over celite. The filter was rinsed with methanol (4 ml x 2.5 vol) and evaporated to 5 volumes. The pale yellow solution was added to 1 N HCl (6 mL, 4 vol), extracted with EtOAc (6 mL, 4 vol) and separated. The organic phase was extracted with 2 N HCl (2 vol) and the combined water layer was basified to pH 12-13 with 6 N NaOH (4 ml, 3 vol). The resulting milky solution gave a white solid after stirring at room temperature at 10 °C for 1 hour. The solid was washed with water (1 vol). After filtration and drying in the oven at 30 °C overnight a white solid was recovered (890 mg, 76 %).

### Alternative Stage 3: Synthesis of (+)-[2-methyl-1-phenyl-2-(1-pyrrolidinyl)propyl]amine. Formic acid reduction.

The chiral diamine **5** (1.45 g, 1 wt) was dissolved in formic acid (5 mL, 3.4 vol) and EtOAc (0.5 mL). 10 % Palladium/carbon (300 mg, 10 % wt; Strem Chemicals, 50 % wet) was added and the suspension heated to 100°C. After stirring for 2 hours the reaction was .finished giving the formilated derivative. The suspension was cooled to 40-50 °C and filtered on Sterimat. The Pd/C was washed with EtOAc (5 mL x 2). The solvent was evaporated at 50°C to 3-4 total vol under vacuum, then a solution of 4 N HCl in water (10 mL) was added. The solution was stirred at 100°C for 3 hours, then at room temperature the water was washed with EtOAc (10 mL x 2). EtOH (2 mL) was added and the solution cooled at 10°C then NaOH 30 % was added to pH 12-13, maintaining the temperature at approximately 10 °C. A solid was obtained and after 1 hour filtered and washed with cold water (5 mL x 2). The solid was dissolved in CH₂Cl₂ and dried for 14 hours under vacuum at 25°C giving the desired compound (710 mg, 71%).

### Description 4: 2-(Methyloxy)-N-[2-methyl-1-phenyl-2-(1-pyrrolidinyl)propyl]-4,6-bis(trifluoromethyl)benzamide chiral

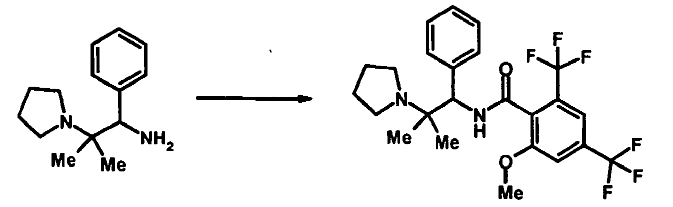

To a solution of diisopropylethylamine (0.915ml; 5.37mmol), 2,4-ditrifluoromethyl-6-methoxy-benzoic acid (0.511g; 1.78mmol) and (+)-[2-methyl-1-phenyl-2-(1-pyrrolidinyl)propyl]amine D3 (0.501g; 1.74mmol) in DMF (50ml) under argon was added HATU (0.676g; 1.78mmol) portionwise. After stirring at room temperature for 3h., followed by standing for ca. 2 days the reaction mixture was purified using an SCX column (an ion exchange column) and the resulting product partitioned between ethyl acetate and water. The solvent was removed in vacuo to afford the title product. ¹H NMR (CDCl₃) δ:0.94 (6H, s), 1.60 - 1.80 (4H, m), 2.55 - 2.75 (4H, m), 3.89 (3H, s), 4.78 (1 H, s), 7.20 -7.40 (7H, m), 7.52 (1H, s). Mass Spectrum (Electrospray LC/MS): Found 489 (MH⁺). C₂₄H₂₆F₆N₂0₂ requires 488. Ret. time 2.06min. Conversion of the title product to the corresponding hydrochloride salt afforded an off-white solid (0.893g; 96%). Due to the use of the chiral intermediate (D3), it is believed that the title product was obtained as a chiral compound.

### Description 4a: Alternative steps for R-(-)-2-(methyloxcy)-N-[2-methyl-1-phenyl-2-(1-pyrrolidinyl)propyl]-4,6-bis(trifluoromethyl)benzamide

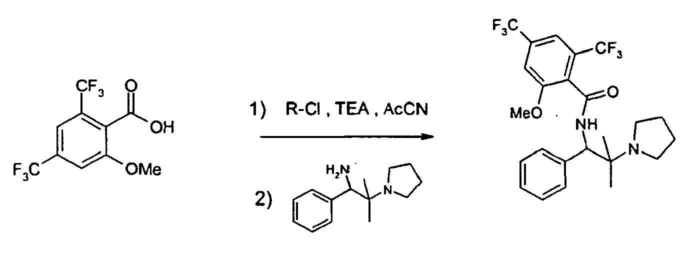

R= CH₃SO₂ - , p-MePhSOz- , (EtO)₂PO-

### (i) Mesylchloride

2,4-ditrifluoromethyl-6-methoxy-benzoic acid (150 mg , 0.55 mmol) was suspended in AcCN (1.5 ml, 10 vol). TEA (0.1 ml, 1.4 eq) was added and the mixture cooled to 0°C. Mesylchloride (0.054 ml, 0.7 mmol) was added and the mixture stirred for 30 min. (+)-[2-methyl-1-phenyl-2-(1-pyrrolidinyl)propyl]amine (100 mg, 0.5 mmol) was added. After stirring for 15 min, TEA (0.1 ml, 1.4 eq) was added and the obtained suspension stirred for 10 min. Methanol (0.2 ml) was added and after stirring for 30 min, the solvent was partially evaporated and the obtained suspension was diluted with EtOAc (2 ml) washed with water (3 ml), a 0.1% bicarbonate solution, water and evaporated to give the title compound (200 mg, ∼80% yield).

### (ii) Tosylchloride

2,4-ditrifluoromethyl-6-methoxy-benzoic acid (300 mg, 1.1 mmol) was suspended in AcCN (3 ml, 10 vol) . TEA (0.2 ml, 1.4 eq) was added and the mixture cooled to 0°C. Tosylchloride (200 mg, 0.7 mmol) was added and the mixture stirred for 30 min at 0°C. (+)-[2-methyl-1-phenyl-2-(1-pyrrolidinyl)propyl]amine (200 mg, 1 mmol) was added . After stirring for 5 min, TEA (0.2 ml, 1.4 eq) was added and the obtained suspension stirred for 30 min at 0°C. The solvent was partially evaporated and the obtained suspension was diluted with EtOAc (5 ml) washed with water (5 ml), 1 M NaOH in water (2 x 5 ml), water (5 ml) and evaporated to give the title compound (500 mg , 90% a/a purity, ∼70% yield ).

### (iii) Diethylchlorophosphate

2,4-ditrifluoromethyl-6-methoxy-benzoic acid (1.58 g, 5.5 mmol) was suspended in AcCN (15 ml, 10 vol). TEA (1.4 ml, 10 mmol) was added and the mixture cooled to 0°C. Diethylchlorophosphate (0.8 ml , 5.5 mmol) was added over 5 min and the mixture stirred for 1 hr 30 min. In a different flask (+)-[2-methyl-1-phenyl-2-(1-pyrrolidinyl)propyl]amine (1.92 g, 5 mmol) was dissolved in CH₂Cl₂ (20 ml, 10 vol) and treated with 1 M NaOH (10 ml). The organic phase was separated, washed with water and evaporated to -10 ml total volume. The CH₂Cl₂ solution was added over 15 min to the activated acid solution cooled at -5°C. It was washed with 5 ml CH₂Cl₂. It was left for another 15 minutes at -5 °C then the solvent was partially evaporated to approximately 10 vol of total. EtOAc (20 ml) was added, washed with 1 M NaOH (2 x 15 ml) , water (2 x 15 ml) and then evaporated to give the title compound (2.33 g, 94%yield).

### (iv) Mesylchloride + amine chiral salt

2,4-ditrifluoromethyl-6-methoxy-benzoic acid (1.58 g, 5.5 mmol) was suspended in AcCN (15 ml, 10 vol) at -10°C. TEA (0.8 ml, 0.5 vol) was added and the mixture cooled to -10°C for 5 min. Mesylchloride (0.42 ml, 0.25 vol) was added and the mixture stirred for 15 min. CH₂Cl₂ (15 ml) was added and cooled to -15°C. TEA (2 ml) was added followed by (+)-[2-methyl-1-phenyl-2-(1-pyrrolidinyl)propyl]amine (1.9 g, 5 mmol) and CH₂Cl₂ (15 ml). The reaction temperature increased to 5°C, after stirring at this temperature for 30 min the reaction was terminated. The solvent was partially evaporated and the obtained suspension was diluted with EtOAc (20 ml) washed with 1 M NaOH (2 x 20 ml), water (2 x 20 ml). The ethyl acetate was filtered and evaporated to give the title compound (2.4g, 94%)

### (v) Tosylchloride + amine chiral salt

2,4-ditrifluoromethyl-6-methoxy-benzoic acid (1.7 g, 6 mmol) was suspended in AcCN (17 ml, 10 vol) and the mixture cooled to -10°C. TEA (0.77 ml, 5 mmol) was added then tosylchloride (1 g), and the mixture stirred for 20 min. TEA (1.6 ml) was added followed by (+)-[2-methyl-1-phenyl-2-(1-pyrrolidinyl)propyl]amine (1.9 g, 5 mmol) and CH₂Cl₂ (10 ml). The reaction temperature increased to 0 °C, after stirring at this temperature for 30 min the reaction was terminated. The solvent was partially evaporated to approximately 5 vol, and the obtained suspension was diluted with EtOAc (20 ml), washed with 1 M NaOH (2 x 20 ml), then by water-NaOH-NaOH, water (2 x 20 ml). The ethyl acetate was filtered and evaporated to give the title compound (2 g , 80% a/a purity, ∼70% yield ).

### (vi) Diethylchlorophosphate + amine chiral salt

2,4-ditrifluoromethyl-6-methoxy-benzoic acid (1.58g, 5.5mmol) was suspended in AcCN (15 ml, 10 vol). TEA (1.4ml, 10mmol) was added and the mixture cooled to 0°C. Diethylchlorophosphate (0.8ml , 5.5mmol) was added over 5 min and the mixture stirred for 1 hr 30 min. The mixture was cooled to -20°C, CH₂Cl₂ (10 ml) was added at -20°C followed by (+)-[2-methyl-1-phenyl-2-(1-pyrrolidinyl)propyl]amine (1.92 g, 5 mmol) followed by CH₂Cl₂ (5 ml). After stirring for 5 min, TEA (0.8 ml) was added and after stirring at -10°C for 1 hr 30min the reaction was terminated. The solvent was partially evaporated to approximately 10 vol, EtOAc (20 ml) was added , washed with 1 M NaOH (2 x 15 ml), water (2 x 15 ml) and then evaporated to give the title compound (2.3 g, 96% a/a purity, ∼90%yield )

### Description 5: 2-(Methyloxy)-N-[2-methyl-1-phenyl-2-(1-pyrrolidinyl)propyl]-4,6-bis(trifluoromethyl)benzamide hydrochloride chiral - alternative method

### Step 1: (±)[2-Methyl-1-phenyl-2-(1-pyrrolidinyl)propyl]amine (D2)

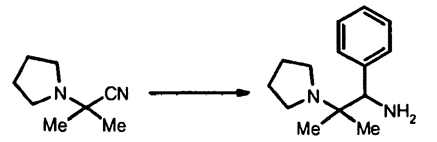

To a solution of 2-methyl-2-(1-pyrrolidinyl)propanenitrile D1 (40g; 289.85mmol) in dry THF (0.8L) under nitrogen, cooled at -78°C, was added dropwise a solution of phenyl lithium in dibutyl ether over 40 minutes (305.1mL of a 1.9M solution; 579.70mmol). After 2h the reaction was allowed to reach room temperature and then stirred overnight at this temperature. The mixture was quenched at 0°C with a saturated solution of NaHCO₃ (0.8L) and stirred for 15 minutes and diluted with water (ca.0.6L). The phases were separated and the aqueous back extracted with diethylether (2x1L). The collected organics were dried over Na₂SO₄ and evaporated in vacuo to get 90g of crude material as a yellow oil that was dissolved in methanol (1 L) at 0°C and treated portionwise with sodium borohydride (21.93g; 579.70mmol). After 1 hour at 0°C and then overnight at room temperature the mixture was cooled and quenched with water (ca.0.5L). Methanol was evaporated in vacuo and the aqueous phase, diluted with water (200mL), was extracted with DCM (3x800mL).

The collected organics were dried over Na₂SO₄ and evaporated in vacuo to get the title product (51g) as a yellow solid, used in step 2 without further purification.

### Step 2: [2-Methyl-1-phenyl-2-(1-pyrrolidinyl)propyl]amine R(-)a methoxy phenyl acetic acid salt

[2-Methyl-1-phenyl-2-(1-pyrrolidinyl)propyl]amine D2 from step 1 (51g; 234mmol) was dissolved in isopropanol (0.765L, 15 volumes, relative volumes being referred to the quantity of [2-methyl-l-phenyl-2-(l-pyrrolidinyl)propyl]amine). To this stirred solution, heated at 50°C, was added a solution of R(-)a methoxy phenyl acetic acid (38.83g; 234mmol) in isopropanol (0.255L, 5 volumes, relative volumes being referred to the quantity of [2-methyl-1-phenyl-2-(1-pyrrolidinyl)propyl]amine). After 1.5h the mixture was cooled to room temperature and then left stirring at this temperature overnight. The solid was recovered by filtration and washed with cold isopropanol. This solid (40.5g) was suspended in isopropanol (0.648L, 16 volumes, relative volumes being referred to the quantity of solid obtained in the last filtration step), and heated at 60°C for 2h, at room temperature overnight and recovered by filtration. This solid (38.5g) was suspended in isopropanol (0.616L, 16 volumes, relative volumes being referred to the quantity of solid obtained in the last filtration step), and heated at 60°C for 2h and at room temperature overnight, then recovered by filtration. This solid (37.8g) was suspended in isopropanol (0.756L, 20 volumes, relative volumes being referred to the quantity of solid obtained in the last filtration step), and heated at 60°C for 2h and at room temperature overnight, then recovered by filtration. This solid (36.5g) was suspended in isopropanol (0.912L, 25 volumes, relative volumes being referred to the quantity of solid obtained in the last filtration step), and heated at 60°C for 2h and then filtered at room temperature. This solid (34g) was suspended in isopropanol (0.850L, 25 volumes, relative volumes being referred to the quantity of solid obtained in the last filtration step), and heated at 60°C for 2h and filtered at room temperature. This solid (31.5g) was suspended in isopropanol (0.787L, 25 volumes, relative volumes being referred to the quantity of solid obtained in the last filtration step), and heated at 60°C for 2h, cooled down to 40°C and then filtered to get the title material (27g) as a white solid.

### Step 3: 2-(Methyloxy)-4,6-bis(trifluoromethyl)benzoyl chloride

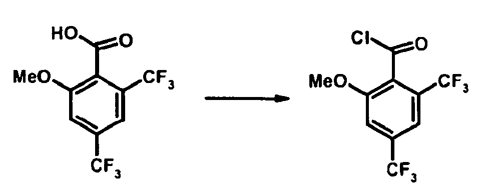

To a solution of 2-(methyloxy)-4,6-bis(trifluoromethyl)benzoic acid (20.2g; 70.14mmol) in dry DCM (400mL), at 0°C, was added dropwise oxalyl chloride (13.4mL; 154.31mmol) followed by dry DMF (5 drops). The reaction was allowed to reach room temperature. After overnight stirring the solvent was evaporated in vacuo to get the title product (23.5g) as a yellow slurry used without further purification.

### Step 4: 2-(Methyloxy)-N-[2-methyl-1-phenyl-2-(1-pyrrolidinyl)propyl]-4,6-bis(trifluoromethyl)benzamide chiral (E15)

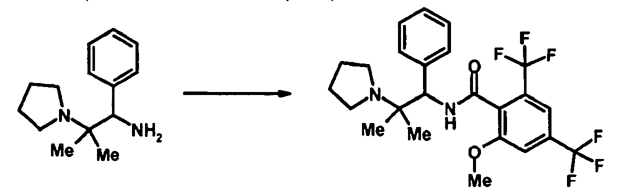

[2-Methyl-1-phenyl-2-(1-pyrrolidinyl)propyl]amine R(-)a methoxy phenyl acetic acid salt from step 2 (22g; 57.3mmol) was suspended in DCM at 0°C, treated with 1M NaOH solution (86mL) and stirred at room temperature for 20 minutes. To the mixture water (250mL) was added, the phases separated and the aqueous one extracted with DCM (2x300mL). The collected organics were dried over Na₂SO₄ and evaporated in vacuo to get 12.3g of white solid that was diluted with dry DCM (200mL) under nitrogen and cooled at 0°C. To this solution was added triethylamine (23.92mL; 172mmol) and a solution of 2-(methyloxy)-4,6-bis(trifluoromethyl)benzoyl chloride from Step 3 in dry DCM (190ml of a 200mL solution in DCM of the step 3 material) over 30 minutes. The reaction was left stirring at room temperature for 2 hours and then quenched with a saturated solution of NaHCO₃ (ca.450mL). The phases were separated and the organic one washed with water (500mL), dried over Na₂SO₄ and evaporated in vacuo to get crude material that was purified by silica gel flash chromatography eluting with DCM/methanol 97/3. Evaporation of the solvent afforded the title material (26g) as a pale yellow solid.

### Step 5: 2-(Methyloxy)-N-[2-methyl-1-phenyl-2-(1-pyrrolidinyi)propyl]-4,6-bis(trifluoromethyl)benzamide hydrochloride chiral

2-(Methyloxy)-*N*-[2-methyl-1-phenyl-2-(1-pyrrolidinyl)propyl]-4,6-bis(trifluoromethyl) benzamide from step 4 (10g; 20.47mmol) was dissolved in dry ethyl ether (200mL), cooled to 0°C and treated with 1 M solution of HCl in ethyl ether (21.5mL; 21.49mmol). After 0.5h the solid was collected by filtration, washed with diethyl ether and dried at 45°C overnight to get the title material (9.1 g) as a pale yellow solid. Due to the use of the chiral intermediate made in step 2, it is believed that the title product was obtained as a chiral compound.

In a different batch, 2-(methyloxy)-*N*-[2-methyl-1-phenyl-2-(1-pyrrolidinyl)propyl]-4,6-bis(trifluoromethyl)benzamide hydrochloride was made using the chiral intermediate: (+)-[2-methyl-1-phenyl-2-(1-pyrrolidinyl)propyl]amine, and the specific rotation of the resulting compound was determined to be -32.6°.

In a separate crystal structure study, the absolute configuration of (-)-2-(methyloxy)-*N*-[2-methyl-1-phenyl-2-(1-pyrrolidinyl)propyl]-4,6-bis(trifluoromethyl)benzamide was determined to be R.

### Example 1: R-(-)-2-(Methyloxy)-N-[2-methyl-1-phenyl-2-(1-pyrrolidinyl)propyl]-4,6-bis(trifluoromethyl)benzamide monosuccinate

### Method (a)

4.84g of 2-(Methyloxy)-*N*-[2-methyl-1-phenyl-2-(1-pyrrolidinyl)propyl]-4,6-bis(trifluoromethyl)benzamide hydrochloride, prepared according to Description 5, were treated with 150mL of 2M solution of NaHCO₃ and 250mL of EtOAc. The mixture was stirred for 20 minutes and then the aqueous phase was extracted with EtOAc. Combined organic phases were dried over Na₂SO₄ and evaporated to dryness to get 2-(methyloxy)-*N*-[2-methyl-1-phenyl-2-(1-pyrrolidinyl)propyl]-4,6-bis(trifluoromethyl)benzamide (free base) 4.5g as a white solid.

300.3mg of the 2-(methyloxy)-*N*-[2-methyl-1-phenyl-2-(1-pyrrolidinyl)propyl]-4,6-bis(trifluoromethyl)benzamide and 73mg (1 eq) of succinic acid were dissolved in 4ml EtOAc with stirring, sonication and heating. 2ml heptane was then added dropwise to the solution with stirring. A gummy solid began to form, so this was taken into solution with heating. On cooling to ambient, solid began precipitating from solution. The slurry thickened considerably over the next hour, and was allowed to stir overnight at ambient. The solid was then isolated by filtration and dried overnight *in vacuo* at 40°C: 322.26mg of the title compound was recovered after drying (86% th).

### Method (b) (alternative, scale up method)

### Stage 1

A mixture of pyrrolidine (1wt) and acetone (1 vol) in toluene (4 vol) was cooled to 0°C -6°C. A solution of potassium cyanide (1wt) in water (5 vol) was added portionwise maintaining the temperature below 6°C. The mixture was stirred for 1.5 hr to 2 hrs at 0°C -6°C.. The two phase were separated and the organic one washed with a brine solution (4 vol). The combined aqueous phases from above were extracted with toluene (8 vol). The combined organic phases were filtered on a magnesium sulphate pad under pressure (min 1 hour) to give 2-methyl-2-(1-pyrrolidinyl)propanenitrile (D3 above). The solution was cooled to -60°C to -50°C. A solution of 1.9 M phenyllitium in dibuthylether (8.9 wt) was added keeping the temperature below -50°C. After the addition, the mixture was stirred for additional 1 to 1.5 hrs at -60° to -50°C, and then it was warmed to 15 to 25°C. Acetone (1.5 vol) was added allowing the exotherm to increase the contents temperature up to a maximum of 40°C. The mixture was cooled to 20°C to 26°C and water (10 vol) was added. The phases were stirred for 30-60 min and then separated. The organic layer was washed with a brine solution (10 vol) and evaporated to dryness. The crude was then dissolved in methanol (11 vol) and the solution cooled to 0°C to 6°C. A solution of NaBH₄ (0.4wt) in aqueous 1 M NaOH (3.2 vol) was added maintaining the temperature between 0°C to 6°C. Water (2 vol) was added. The reaction mixture was stirred for 1-2 hrs at 0-6°C. Water (3.5 vol) was added to the mixture, maintaining the contents below 10°C, followed by acetic acid (2.5 wt), maintaining the contents below 15°C. The mixture was stirred for 15 to 30 minutes then further water (5 vol) was added maintaining the contents below 15°C. The mixture was stirred for 15-30 min, maintaining continuously below 15°C and then the two phases separated. A solution of aqueous 1 M NaOH (about 4 vol) was added to the aqueous phase, maintaining the contents below 30°C to adjust the pH >12. After 30-60 min stirring, toluene (8 vol) was added and the two phases separated. The aqueous phase was extracted with toluene (2 vol). A 1M HCl aqueous solution (10 vol) was added to the combined organic layers maintaining the contents temperature below 30°C. After 15-30 min of stirring, the two phases were separated. The aqueous layer was washed two times with toluene (2x4 vol). A solution of NaOH 1 M aqueous (3.5 vol) was added to the aqueous layer maintaining the contents temperature below 30°C, to adjust the pH to >12. The resulting suspension was stirred for 30-60 min and then was filtered. The cake was washed with water (2 vol), to give 2-[methyl-1-phenyl-2-(1-pyrrolidinyl)propyl]amine (D2 above). The crude compound was suspended in water (17 vol) and the mixture was heated to 30°C and stirred at this temperature for 1-2 hrs. It was cooled to 20°C, stirred for 2-3 hrs and filtered. The cake was washed with water (3 vol) and the solid was dried in a vacuum oven at 35°C (yield 65%).

### Stage 2

2-[methyl-1-phenyl-2-(1-pyrrolidinyl)propyl]amine (20 g; 1 wt) was dissolved in acetone (15 vol) and the mixture was stirred for 15 min under nitrogen, heating to 40 °C. 15% of a solution of *R*-(-)-methoxyphenylacetic acid (0.726 wt) in acetone (5 vol.) was added and stirred for 15 min at 40°C. After this, [2-methyl-1-phenyl-2-(1-pyrrolidinyl)propyl]amine R(-)a methoxy phenyl acetic acid salt was added, as a seed. The rest of the acid solution was added over 30 min and it was left for the salt to precipitate, keeping the temperature to 40°C and stirring constantly for 1 hr. The suspension was cooled down to room temperature and was left stirring overnight. The solid was filtered and washed with cold acetone (2 x 2 vol). The solid was dried in vacuum oven at 40 °C overnight to give [2-methyl-1-phenyl-2-(1-pyrrolidinyl)propyl]amine R(-)a methoxy phenyl acetic acid salt (yield 49%).

### Stage 3

2-Methyl-1-phenyl-2-(1-pyrrolidinyl)propyl]amine R(-)a methoxy phenyl acetic acid salt (9.1 g,1 wt) was suspended in DCM (91 ml, 10 vol) and a solution of NaOH 1 M (5 vol, 45 ml) was added. After 15 min, the two phases were separated. The aqueous layer was extracted with DCM (45 ml, 5 vol). The combined organic phases were concentrated to dryness and dissolved with fresh DCM (3 vol, 27 ml). TBTU (9.11 g, 1 wt), 2-(methyloxy)-4,6-bis(trifluoromethyl)benzoic acid (6.82 g) and triethylamine (344 ml, 0.433 vol) in acetonitrile (45 ml, 5 vol) was stirred at room temperature for 2 hour 40 min and the free base in DCM prepared above was added in 5 minutes. The reaction mixture was stirred for 2 hr 30 min at room temperature.

The reaction mixture was concentrated to 3 vol (27 ml) and fresh ethyl acetate (11 vol, 100 ml). was added. The organic phase was washed with 15% sodium carbonate aqueous solution (4 vol, 36 ml) and water (4 vol, 36 ml). The organic layer was washed with water (1×5 vol + 2×3 vol) and concentrated under vacuum to 4 vol. Further ethyl acetate was added (4 vol, 36 ml). The organic phase was concentrated again to 4 vol. Fresh ethyl acetate was added (7 vol, 63.77 ml). The solution was heated to 70°C and succinic acid (2.25g) was added. After 20 min a seed (final compound) was added. The reaction mixture was stirred for 20 min more at 70°C and then cooled to room temperature and stirred at room tempearutre. The suspension was filtered, the cake washed with ethyl acetate (1x 2 vol) and the solid dried in a vacuum oven, to give 2-(methyloxy)-*N*-[2-methyl-1-phenyl-2-(1-pyrrolidinyl)propyl]-4,6-bis(trifluoromethyl)benzamide monosuccinate (7.87g, 67%).

### Stage 4

2-(methyloxy)-*N*-[2-methyl-1-phenyl-2-(1-pyrrolidinyl)propyl]-4,6-bis(trifluoromethyl)benzamide monosuccinate (317.63 g, 1 wt) was dissolved in DCM (1588 ml, 5 vol) at room temperature and the solution was stirred for 15 min and filtered. IPA (6.3 vol) was added and the mixture was concentrated to 8 vol. Then a further 3.7 vol was added and distilled again until 8 vol. The mixture was distilled at reflux until 8 vol. The mixture was cooled to 25°C over 90 minutes. 2-(methyloxy)-*N*-[2-methyl-1-phenyl-2-(1-pyrrolidinyl)propyl]-4,6-bis(trifluoromethyl)benzamide monosuccinate (0.001 wt, 0.3 g), as the seed, was added at 65°C. The suspension was stirred overnight at room temperature and then filtered and washed with IPA (600 ml, 2 vol) and the solid dried in a vacuum oven at 40°C overnight. Yield 84%.

### Example 2: R-(-)-2-(Methyloxy)-N-[2-methyl-1-phenyl-2-(1-pyrrolidinyl)propyl]-4,6-bis(trifluoromethyl)benzamide 1,5-heminapadisylate

300.4mg of the 2-(methyloxy)-*N*-[2-methyl-1-phenyl-2-(1-pyrrolidinyl)propyl]-4,6-bis(trifluoromethyl)benzamide prepared as described in Example 1 was dissolved in 4ml IPA with stirring, sonication and heating. 114mg of 1,5-naphthalenedisulfonic acid was dissolved in 1 ml IPA, and the 2 solutions were combined. A thick gum formed rapidly, so this was taken into solution with heating. On cooling to ambient, solid began precipitating from solution. The slurry thickened considerably over the next hour, and was allowed to stir overnight at ambient. The solid was then isolated by filtration and dried overnight *in vacuo* at 40°C, and then for a further night at 60°C. 343.95mg of the title compound was recovered after drying (83% th).

**Table 1: XRPD angles and d spacings for R-(-)-2-(Methyloxy)-N-[2-methyl-1-phenyl-2-(1-pyrrolidinyl)propyl]-4,6-bis(trifluoromethyl)benzamide monosuccinate**

| **2θ/°** | **d-spacing / Å** |
|---|---|
| 8.0 | 11.1 |
| 8.8 | 10.1 |
| 10.6 | 8.3 |
| 11.1 | 7.9 |
| 11.2 | 7.9 |
| 12.5 | 7.1 |
| 13.2 | 6.7 |
| 14.0 | 6.3 |
| 14.4 | 6.2 |
| 15.9 | 5.6 |
| 16.1 | 5.5 |
| 16.6 | 5.3 |
| 17.2 | 5.1 |
| 17.4 | 5.1 |
| 17.6 | 5.0 |
| 17.9 | 4.9 |
| 18.4 | 4.8 |
| 18.6 | 4.8 |
| 18.8 | 4.7 |
| 19.7 | 4.5 |
| 20.3 | 4.4 |
| 21.3 | 4.2 |
| 22.3 | 4.0 |
| 22.6 | 3.9 |
| 22.9 | 3.9 |
| 23.2 | 3.8 |
| 23.9 | 3.7 |
| 24.6 | 3.6 |
| 25.2 | 3.5 |
| 25.5 | 3.5 |
| 26.5 | 3.4 |

**Table 2: XRPD angles and d spacings for R-(-)-2-(Methyloxy)-N-[2-methyl-1-phenyl-2-(1-pyrrolidinyl)propyl]-4,6-bis(trifluoromethyl)benzamide 1,5-heminapadisylate**

| **2θ/ °** | **d-spacing / Å** |
|---|---|
| 8.0 | 11.0 |
| 8.9 | 10.0 |
| 9.3 | 9.5 |
| 9.7 | 9.1 |
| 11.3 | 7.9 |
| 11.6 | 7.6 |
| 11.9 | 7.4 |
| 14.4 | 6.2 |
| 14.6 | 6.1 |
| 14.9 | 5.9 |
| 15.3 | 5.8 |
| 15.8 | 5.6 |
| 16.0 | 5.5 |
| 16.6 | 5.3 |
| 17.2 | 5.1 |
| 17.9 | 5.0 |
| 18.2 | 4.9 |
| 19.4 | 4.6 |
| 19.9 | 4.5 |
| 20.8 | 4.3 |
| 21.1 | 4.2 |
| 21.4 | 4.1 |
| 21.7 | 4.1 |
| 22.2 | 4.0 |
| 22.6 | 3.9 |
| 23.2 | 3.8 |
| 24.1 | 3.7 |
| 24.5 | 3.6 |
| 24.9 | 3.6 |
| 25.3 | 3.5 |
| 26.3 | 3.4 |
| 29.4 | 3.0 |

### Description of Figures

Figure 1: X-Ray powder diffraction data obtained for R-(-)-2-(Methyloxy)-*N*-[2-methyl-1-phenyl-2-(1-pyrrolidinyl)propyl]-4,6-bis(trifluoromethyl)benzamide succinate. Characteristic peaks for the solid state form are summarised in Table 1 with and calculated lattice spacings. Peak positions were measured using Highscore software.
Figure 2: X-Ray powder diffraction data obtained for R-(-)-2-(Methyloxy)-*N*-[2-methyl-1-phenyl-2-(1-pyrrolidinyl)propyl]-4,6-bis(trifluoromethyl)benzamide napadisylate. Characteristic peaks for the solid state form are summarised in Table 1 with and calculated lattice spacings. Peak positions were measured using Highscore software.

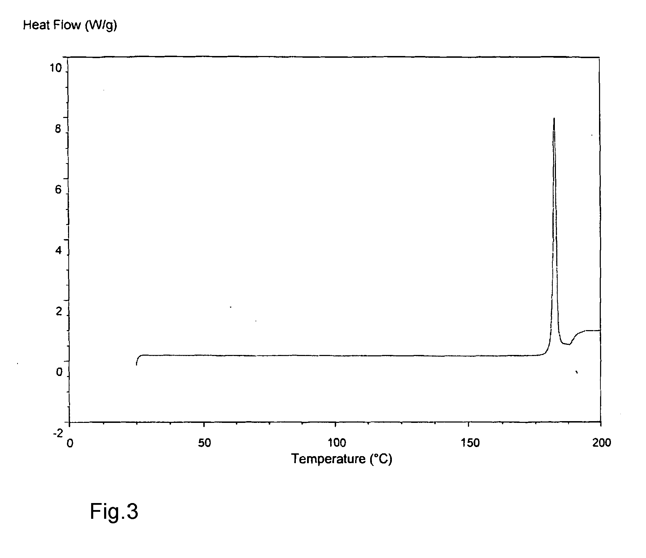

Figure 3: Differential Scanning Calorimetry (DSC) thermogram of R-(-)-2-(Methyloxy)-*N-*[2-methyl-1-phenyl-2-(1-pyrrolidinyl)propyl]-4,6-bis(trifluoromethyl)benzamide succinate. The sample shows an endotherm, with onset temp=181°C, due to melt event.

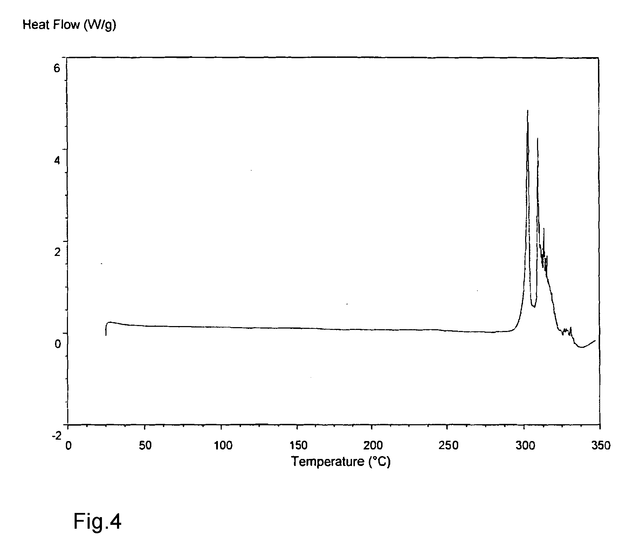

Figure 4: Differential Scanning Calorimetry (DSC) thermogram of R-(-)-2-(Methyloxy)-*N*-[2-methyl-1-phenyl-2-(1-pyrrolidinyl)propyl]-4,6-bis(trifluoromethyl)benzamide napadisylate. The sample shows an endotherm with onset temp=301°C, due to melt event. Minor endotherms are also observed with onset temp=159°C, possibly due to solvent loss or melt event and at onset temp=225°C, possibly due to a melt event.

### X-Ray Powder Diffraction

The data were acquired on a PANalytical X'Pert Pro^{™} powder diffractometer, model PW3040/60, serial number DY1850 using an XCelerator^{™} detector. The acquisition conditions were: radiation: Cu Kα, generator tension: 40 kV, generator current: 45 mA, start angle: 2.0°2θ, end angle: 40.0°2 θ, step size: 0.0167°2 θ, time per step: 31.75 seconds. The sample was prepared by mounting a few milligrams of sample on a Si wafer (zero background) plates, resulting in a thin layer of powder.

It will be recognised that spectra and diffraction data will vary slightly according to various factors such as the temperature, concentration and instrumentation used. The skilled person will recognise that XRPD peak positions are affected by differences in sample height. The peak positions quoted herein are thus subject to a variation of +/- 0.15 degrees 2-theta.

It should be noted that more than one polymorph of the salt may have existed in the samples tested. This may have caused some of the minor peaks in the XRPD reading. A skilled person would understand that such minor peaks may not appear in an XRPD reading for a sample containing only one polymorph.

### Differential Scanning Calorimetry (DSC)

DSC thermograms were obtained using a TA Q1000 calorimeter, serial number 1000-0126. The samples were weighed into an aluminium pan, a pan lid placed on top and lightly crimped without sealing the pan. The experiment was conducted using a heating rate of 10°C min⁻¹.

## Claims

1. 2-(Methyloxy)-*N*-[2-methyl-1-phenyl-2-(1-pyrrolidinyl)propyl]-4,6-bis(trifluoromethyl)benzamide succinate or a solvate thereof.

2. 2-(Methyloxy)-*N*-[2-methyl-1-phenyl-2-(1-pyrrolidinyl)propyl]-4,6-bis(trifluoromethyl)benzamide napadisylate or a solvate thereof.

3. A compound as claimed in claim 1, which is 2-(methyloxy)-*N*-[2-methyl-1-phenyl-2-(1-pyrrolidinyl)propyl]-4,6-bis(trifluoromethyl)benzamide monosuccinate or a solvate thereof.

4. A compound as claimed in claim 1, which is R-(-)-2-(methyloxy)-*N*-[2-methyl-1-phenyl-2-(1-pyrrolidinyl)propyl]-4,6-bis(trifluoromethyl)benzamide monosuccinate or a solvate thereof.

5. A compound as claimed in claim 1, which is S-(+)-2-(methyloxy)-*N*-[2-methyl-1-phenyl-2-(1-pyrrolidinyl)propyl]-4,6-bis(trifluoromethyl)benzamide monosuccinate or a solvate thereof.

6. A compound as claimed in claim 2 which is 2-(methyloxy)-*N*-[2-methyl-1-phenyl-2-(1-pyrrolidinyl)propyl]-4,6-bis(trifluoromethyl)benzamide-1,5-napadisylate or a solvate thereof.

7. A compound as claimed in claim 2 which is 2-(methyloxy)-*N*-[2-methyl-1-phenyl-2-(1-pyrrolidinyl)propyl]-4,6-bis(trifluoromethyl)benzamide-1,5-heminapadisylate or a solvate thereof.

8. A compound as claimed in claim 2, which is R-(-)-2-(methyloxy)-*N-*[2-methyl-1-phenyl-2-(1-pyrrolidinyl)propyl]-4,6-bis(trifluoromethyl)benzamide 1,5-heminapadisylate or a solvate thereof.

9. A compound as claimed in claim 2, which is S-(+)-2-(methyloxy)-*N*-[2-methyl-1-phenyl-2-(1-pyrrolidinyl)propyl]-4,6-bis(trifluoromethyl)benzamide 1,5-heminapadisylate or a solvate thereof.

10. A compound as claimed in any of claims 1-9, in crystalline form.

11. A compound as claimed in claim 1 having an X-ray powder diffraction pattern, wherein said X-ray powder diffraction pattern comprises 2 theta angles at one or more positions selected from the group consisting of 10.6±0.1, 11.1±0.1 and 21.3±0.1 degrees.

12. A compound as claimed in claim 1 having an XRPD diffractogram substantially as illustrated in Figure 1.

13. A compound as claimed in claim 1 having an XRPD diffractogram with signals substantially as listed in Table 1.

14. A compound as claimed in claim 2 having an XRPD diffractogram substantially as illustrated in Figure 2.

15. A compound as claimed in claim 2 having an XRPD diffractogram with signals substantially as listed in Table 2.

16. A compound as claimed in any of claims 1-15 for use in therapy.

17. A compound as claimed in any of claims 1-15 for use in the treatment of a disorder mediated by GlyT1.

18. A compound as claimed in any of claims 1-15, for use in the treatment of psychosis.

19. A compound as claimed in any of claims 1-15, for use in the treatment of schizophrenia, dementia or attention deficit disorder.

20. Use of a compound as claimed in any of claims 1-15 in the preparation of a medicament for the treatment of a disorder mediated by GlyT1.

21. Use of a compound as claimed in any of claims 1-15 in the preparation of a medicament for the treatment of psychosis.

22. The use as claimed in claim 21, wherein the psychosis is schizophrenia, dementia or attention deficit disorder.

23. A pharmaceutical composition comprising a compound as claimed in any of claims 1-15 and at least one pharmaceutically acceptable carrier, diluent or excipient.

24. A pharmaceutical composition as claimed in claim 23 further comprising one or more other therapeutic agents, selected from antidepressant agents (selected from 5HT3 antagonists, serotonin agonists, NK-1 antagonists, selective serotonin reuptake inhibitors (SSRI), noradrenaline re-uptake inhibitors (SNRI), tricyclic antidepressants, dopaminergic antidepressants, H3 antagonists, 5HT1A antagonists, 5HT1B antagonists, 5HT1D antagonists, D1 agonists, M1 agonists); anticonvulsant agents; atypical antipsychotic drugs and cognitive enhancers.

## Patentansprüche

1. 2-(Methyloxy)-N-[2-methyl-1-phenyl-2-(1-pyrrolidinyl)propyl]-4,6-bis(trifluormethyl)-benzamidsuccinat oder ein Solvat davon.

2. 2-(Methyloxy)-N-[2-niethyl-1-phenyl-2-(1-pyrrolidinyl)propyl]-4,6-bis(trifluornlethyl)-benzamidnapadisylat oder ein Solvat davon.

3. Verbindung wie in Anspruch 1 beansprucht, nämlich 2-(Methyloxy)-N-[2-methyl-l-phenyl-2-(1-pyrrolidinyl)propyl]-4,6-bis(trifluormethyl)benzamidmonosuccinat oder ein Solvat davon.

4. Verbindung wie in Anspruch 1 beansprucht, nämlich R-(-)-2-(Methyloxy)-N-[2-methyl-1-phenyl-2-(1-pyrrolidinyl)propyl]-4,6-bis(trifluomvethyl)benzamidrnonosuccinat oder ein Solvat davon.

5. Verbindung wie in Anspruch 1 beansprucht, nämlich S-(+)-2-(Methyloxy)-N-[2-methyl-1-phenyl-2-(1-pyrrolidinyl)propyl]-4,6-bis(trifluorrnethyl)bcnzamidnionusuccinat oder ein Solvat davon.

6. Verbindung wie in Anspruch 2 beansprucht, nämlich 2-(Methyloxy)-N-[2-methyl-1-phenyl-2-(1-pyirolidinyl)propyl]-4,6-bis(trifluormethyl)benzanlid-1,5-napadisylat oder ein Solvat davon.

7. Verbindung wie in Anspruch 2 beansprucht, nämlich 2-(Methyloxy)-N-[2-tnethyl-1-phenyl-2-(1-pyrrolidinyl)propyl]-4,6-bis(trifluormethyl)benzamid-1,5-heminapadisylat oder ein Solvat davon.

8. Verbindung wie in Anspruch 2 beansprucht, nämlich R-(-)-2-(Methyloxy)-N-[2-methyl-1-phenyl-2-(1-pyrrolidmyl)propyl]-4,6-bis(trifluormethyl)benzamid-1,5-hemmapadisylat oder ein Solvat davon.

9. Verbindung wie in Anspruch 2 beansprucht, nämlich S-(+)-2-(Methyloxy)-N-[2-methyl-1-phenyl-2-(1-pyrrolidinyl)propyl]-4,6-bis(trifluormethyl)benzamid-1,5-heminapadisylat oder ein Solvat davon.

10. Verbindung wie in einem der Ansprüche 1 bis 9 beansprucht, in kristalliner Form.

11. Verbindung wie in Anspruch 1 beansprucht, mit einem Röntgenpulverbeugungsmuster, wobei das Röntgenpulverbeugungsmuster 2 theta-Winkel an einer oder mehreren Positionen, ausgewählt aus der Gruppe, bestehend aus 10,6 ± 0,1, 11,1 ± 0,1 und 21,3 ± 0,1 Grad, umfasst.

12. Verbindung wie in Anspruch 1 beansprucht, mit einem im Wesentlichen wie in Figur 1 dargestellten XRPD-Diffraktogramm.

13. Verbindung wie in. Anspruch 1 beansprucht, mit einem XRPD-Diffraktogramm mit im Wesentlichen wie in Tabelle 1 aufgelisteten Signalen.

14. Verbindung wie in Anspruch 2 beansprucht, mit einem im Wesentlichen wie in Figur 2 dargestellten XRPD-Diffraktogramm.

15. Verbindung wie in Anspruch 2 beansprucht, mit einem XRPD-Diffraktogramm mit im Wesentlichen wie in Tabelle 2 aufgelisteten Signalen.

16. Verbindung wie in einem der Ansprüche 1 bis 15 beansprucht, zur Verwendung in der Therapie.

17. Verbindung wie in einem der Ansprüche 1 bis 15 beansprucht, zur Verwendung bei der Behandlung einer durch GlyT1 vermittelten Störung.

18. Verbindung wie in einem der Ansprüche 1 bis 15 beansprucht, zur Verwendung bei der Behandlung von Psychose.

19. Verbindung wie in einem der Ansprüche 1 bis 15 beansprucht, zur Verwendung bei der Behandlung von Schizophrenie, Demenz oder Aufmerksamkeitsdefizitsyndrom.

20. Verwendung einer Verbindung wie in einem der Ansprüche 1 bis 15 beansprucht, zur Herstellung eines Medikaments zur Behandlung einer durch GlyT1 vermittelten Störung.

21. Verwendung einer Verbindung wie in einem der Ansprüche 1 bis 15 beansprucht, zur Herstellung eines Medikaments zur Behandlung von Psychose.

22. Verwendung wie in Anspruch 21 beansprucht, wobei die Psychose Schizophrenie, Demenz oder Aufmerksamkeitsdefizitsyndrom ist.

23. Arzneimittel, umfassend eine Verbindung wie in einem der Ansprüche 1 bis 15 beansprucht und mindestens ein(en) pharmazeurisch verträglichen(s) Träger, Verdünnungsmittel oder Exzipienten.

24. Arzneimittel wie in Anspruch 23 beansprucht, ferner umfassend ein oder mehrere andere therapeutische Mittel, ausgewählt aus Antidepressiva (ausgewählt aus 5HT3-Antagonisten, Serotonin-Agonisten, NK-1-Antagonisten, selektiven Serotonin-Wiederaufnahmehemmem (SSRI), Noradrenalin-Wiederaufnahmehemmern (SNRI), tricyclischen Antidepressiva, dopaminergenen Antidepressiva, H3-Antagonisten, 5HT1A-Antagonisten, 5HT1B-Antagonisten, 5HT1D-Antagonisten, D1-Agonisten, M1-Agonisten); Antiepileptika; atypischen antipsychotischen Wirkstoffen und Wahrnehmungsverstärkern.

## Revendications

1. Succinate de 2-(méthyloxy)-*N*-[2-méthyl-1-phényl-2-(1-pyrrolidinyl)propyl]-4,6-bis(trifluorométhyl)benzamide ou un de ses produits de solvatation.

2. Napadisylate de 2-(méthyloxy)-*N*-[2-méthyl-1-phényl-2-(1-pyrrolidinyl)propyl]-4,6-bis(trifluorométhyl)benzamide ou un de ses produits de solvatation.

3. Composé suivant la revendication 1, qui est le monosuccinate de 2-(méthyloxy)-*N*-[2-méthyl-1-phényl-2-(1-pyrrolidinyl)propyl]-4,6-bis(trifluorométhyl)benzamide ou un de ses produits de solvatation.

4. Composé suivant la revendication 1, qui est le monosuccinate de R-(-)-2-(méthyloxy)-*N*-[2-méthyl-1-phényl-2-(1-pyrrolidinyl)propyl]-4,6-bis(trifluorométhyl)benzamide ou un de ses produits de solvatation.

5. Composé suivant la revendication 1, qui est le monosuccinate S-(+)-2-(méthyloxy)-*N*-[2-méthyl-1-phényl-2-(1-pyrrolidinyl)propyl]-4,6-bis(trifluorométhyl)benzamide ou un de ses produits de solvatation.

6. Composé suivant la revendication 2, qui est le 1,5-napadisylate de 2-(méthyloxy)-*N*-[2-méthyl-1-phényl-2-(1-pyrrolidinyl)propyl]-4,6-bis(trifluorométhyl)benzamide ou un de ses produits de solvatation.

7. Composé suivant la revendication 2, qui est le 1,5-héminapadisylate de 2-(méthyloxy)-*N*-[2-méthyl-1-phényl-2-(1-pyrrolidinyl)propyl]-4,6-bis(trifluorométhyl)benzamide ou un de ses produits de solvatation.

8. Composé suivant la revendication 2, qui est le 1,5-héminapadisylate de R-(-)-2-(méthyloxy)-*N*-[2-méthyl-1-phényl-2-(1-pyrrolidinyl)propyl]-4,6-bis(trifluorométhyl)-benzamide ou un de ses produits de solvatation.

9. Composé suivant la revendication 2, qui est le 1,5-héminapadisylate de S-(+)-2-(méthyloxy)-*N*-[2-méthyl-1-phényl-2-(1-pyrrolidinyl)propyl]-4,6-bis(trifluorométhyl)-benzamide ou un de ses produits de solvatation.

10. Composé suivant l'une quelconque des revendications 1 à 9, sous forme cristalline.

11. Composé suivant 1a revendication 1, ayant un diagramme de diffraction des rayons X sur poudre, ledit diagramme de diffraction des rayons X sur poudre comprenant des angles 2 thêta à une ou plusieurs positions choisies dans le groupe consistant en 10,6±0,1, 11,1±0,1 et 21,3±0,1 degrés.

12. Composé suivant 1a revendication 1, ayant un diffractogramme de XRPD substantiellement tel qu'illustré sur la figure 1.

13. Composé suivant la revendication 1, ayant un diffractogramme de XRPD avec des signaux substantiellement tels qu'énumérés sur le Tableau 1.

14. Composé suivant la revendication 2, ayant un diffractogramme de XRPD substantiellement tel qu'illustré sur la figure 2.

15. Composé suivant la revendication 2, ayant un diffractogramme de XRPD avec des signaux substantiellement tels qu'énumérés sur le Tableau 2.

16. Composé suivant l'une quelconque des revendications 1 à 15, destiné à être utilisé en thérapeutique.

17. Composé suivant l'une quelconque des revendications 1 à 15, destiné à être utilisé dans le traitement d'un trouble à médiation par GlyT1.

18. Composé suivant l'une quelconque des revendications 1 à 15, destiné à être utilisé dans le traitement de la psychose.

19. Composé suivant l'une quelconque des revendications 1 à 15, destiné à être utilisé dans le traitement de la schizophrénie, de la démence ou du trouble de déficit d'attention.

20. Utilisation d'un composé suivant l'une quelconque des revendications 1 à 15, dans la préparation d'un médicament destiné au traitement d'un trouble à médiation par GlyT1.

21. Utilisation d'un composé suivant l'une quelconque des revendications 1 à 15, dans la préparation d'un médicament destiné au traitement de la psychose.

22. Utilisation suivant la revendication 21, dans laquelle la psychose est la schizophrénie, la démence ou le trouble de déficit d'attention.

23. Composition pharmaceutique comprenant un composé suivant l'une quelconque des revendications 1 à 15, et au moins un support, diluant ou excipient pharmaceutiquement acceptable.

24. Composition pharmaceutique suivant la revendication 23, comprenant en outre un ou plusieurs autres agents thérapeutiques, choisis entre des antidépresseurs (choisis entre des antagonistes de 5HT3, des agonistes de sérotonine, des antagonistes de NK-1, des inhibiteurs d'absorption de sérotonine sélectifs (SSRI), des inhibiteurs de réabsorption de noradrénaline (SNRI), des antidépresseurs tricycliques, des antidépresseurs dopaminergiques, des antagonistes de H3, des antagonistes de 5HT1A, des antagonistes de 5HT1B, des antagonistes de 5HT1D, des agonistes de D1, des agonistes de M1); des agents anticonvulsivants, des médicaments antipsychotiques atypiques et des agents améliorant la cognition.
